# FASCICULE DE BREVET EUROPEEN

(11) **EP 1 861 418 B1**
(45) Date de publication et mention de la délivrance du brevet: **20.01.2010**
(21) Numéro de dépôt: 06709221.3
(22) Date de dépôt: 02.02.2006
(51) Int. Cl.: C07K 14/05, C07K 19/00, C12N 15/11, G01N 33/68, A61K 39/00

(54) **EPITOPES T CD4+ DES ANTIGENES DE LATENCE DE TYPE I ET II DU VIRUS EPSTEIN-BARR APTES A ETRE RECONNUS PAR LA MAJORITE DES INDIVIDUS DE LA POPULATION CAUCASIENNE ET LEURS APPLICATIONS**
T-CD4+-EPITOPE DER LATENZANTIGENE TYP I UND II DES EPSTEIN-BARR-VIRUS, DIE BEI DER MEHRZAHL VON INDIVIDUEN IN DER KAUKASISCHEN BEVÖLKERUNG ERKANNT WERDEN KÖNNEN, UND ANWENDUNGEN DAVON
T CD4+ EPITOPES OF TYPE I AND II LATENCY ANTIGENS OF THE EPSTEIN-BARR VIRUS, WHICH CAN BE RECOGNISED BY THE MAJORITY OF INDIVIDUALS IN THE CAUCASIAN POPULATION AND APPLICATIONS THEREOF

(30) Priorité: 07.02.2005 FR 0501198
(43) Date de publication de la demande: 05.12.2007
(73) Titulaire: Centre National de la Recherche Scientifique, 75016 Paris (FR); COMMISSARIAT A L'ENERGIE ATOMIQUE, 75015 Paris (FR); UNIVERSITE DE LILLE II, 59800 Lille (FR)
(72) Inventeur: PANCRE, Véronique, F-59310 Orchies (FR); AURIAULT, Claude, 55 avenue de Cannes, 06160 Juan les Pins (FR); DEPIL, Stéphane, F-LILLE 59000 (FR); MAILLERE, Bernard, F-78000 Versailles (FR); MORALES, Olivier, F-59000 Lille (FR); MUNIER, Gaetan, 91120 Palaiseau (FR)
(74) Mandataire: Leblois-Préhaud, Hélène Marthe Georgette
(86) Numéro de dépôt international: PCT/FR2006/000229
(87) Numéro de publication internationale: WO 2006/082313

(56) Documents cités:
- EP-A- 1 229 043
- WO-A-2004/001424
- WO-A-2004/007536
- WO-A-2004/041849
- US-A1- 2004 141 995
- JOURNAL OF VIROLOGY., vol. 78, no. 2, janvier 2004 (2004-01), pages 768-778, XP002351152 US THE AMERICAN SOCIETY FOR MICROBIOLOGY. cité dans la demande
- JOURNAL OF IMMUNOLOGY., vol. 169, 2002, pages 1593-1603, XP002351153 US THE WILLIAMS AND WILKINS CO. BALTIMORE. cité dans la demande
- JOURNAL OF VIROLOGY., vol. 75, no. 18, septembre 2001 (2001-09), pages 8649-8659, XP002351154 US THE AMERICAN SOCIETY FOR MICROBIOLOGY. cité dans la demande
- E ADRIAESSENS ET AL.: "A novel dominant-negative mutant form of Epstein-Barr virus latent membrane protein-1 (LMP1) selectively and differentially impairs LMP1 and TNF signaling pathways" ONCOGENE, vol. 23, no. 15, 2004, pages 2681-2693, XP002392318 GB BASINGSTOKE, HANTS
- J A BARWELL ET AL.: "Overexpression, purification, and crystallization of the DNA binding and dimerization domains of the Epstein-Barr virus nuclear antigen 1" JOURNAL OF BIOLOGICAL CHEMISTRY., vol. 270, no. 35, 1 septembre 1995 (1995-09-01), pages 20556-20559, XP002392596 US AMERICAN SOCIETY OF BIOLOCHEMICAL BIOLOGISTS, BIRMINGHAM, AL.

### SEQUENCE LISTING

<110> Centre National de la Recherche scientifique
   Commissariat à l'Energie Atomique
   université de Lille 2
   PANCRE, Véronique
   AURIAULT, Claude
   DEPIL, Stéphane
   MAILLERE, Bernard
   MORALES, Olivier
   MUNIER, Gaétan
<120> Epitopes T CD4+ des antigènes de latence de type I et II du virus Epstein-Barr aptes à être reconnus par la majorité des individus de la population caucasienne et leurs applications.
<130> F644PCT128
<160> 30
<170> PatentIn version 3.3
<210> 1
   <211> 19
   <212> PRT
   <213> Epstein-Barr virus
<400> 1
   Arg Asp Gly Val Arg Arg Pro Gln Lys Arg Pro Ser Cys Ile Gly Cys
   1               5                   10                  15
   Lys Gly Thr
<210> 2
   <211> 26
   <212> PRT
   <213> Epstein-Barr virus
<400> 2
   Arg Pro Phe Phe His Pro Val Gly Glu Ala Asp Tyr Phe Glu Tyr His
   1               5                   10                  15
   Gln Glu Gly Gly Pro Asp Gly Glu Pro Asp
               20                  25
<210> 3
   <211> 22
   <212> PRT
   <213> Epstein-Barr virus
<400> 3
   Asp Gly Gly Arg Arg Lys Lys Gly Gly Trp Phe Gly Lys His Arg Gly
   1 5 10 15
   Gln Gly Gly Ser Asn Pro
   20
<210> 4
   <211> 26
   <212> PRT
   <213> Epstein-Barr virus
<400> 4
   Asn Pro Lys Phe Glu Asn Ile Ala Glu Gly Leu Arg Ala Leu Leu Ala
   1 5 10 15
   Arg Ser His Val Glu Arg Thr Thr Asp Glu
   20 25
<210> 5
   <211> 26
   <212> PRT
   <213> Epstein-Barr virus
<400> 5
   Lys Thr Ser Leu Tyr Asn Leu Arg Arg Gly Thr Ala Leu Ala Ile Pro
   1 5 10 15
   Gln Cys Arg Leu Thr Pro Leu Ser Arg Leu
   20 25
<210> 6
   <211> 24
   <212> PRT
   <213> Epstein-sarr virus
<400> 6
   Pro Gln Cys Arg Leu Thr Pro Leu Ser Arg Leu Pro Phe Gly Met Ala
   1 5 10 15
   Pro Gly Pro Gly Pro Gln Pro Gly
   20
<210> 7
   <211> 23
   <212> PRT
   <213> Epstein-Barr virus
<400> 7
   Met Val Phe Leu Gln Thr His Ile Phe Ala Glu Val Leu Lys Asp Ala
   1               5                   10                  15
   Ile Lys Asp Leu Val Met Thr
               20
<210> 8
   <211> 24
   <212> PRT
   <213> Epstein-Barr virus
<400> 8
   Phe Ala Glu Val Leu Lys Asp Ala Ile Lys Asp Leu Val Met Thr Lys
   1               5                   10                  15
   Pro Ala Pro Thr Cys Asn Ile Arg
               20
<210> 9
   <211> 19
   <212> PRT
   <213> Epstein-Barr virus
<400> 9
   Leu Pro Pro Trp Phe Pro Pro Met Val Glu Gly Ala Ala Ala Glu Gly
   1               5                   10                  15
   Asp Asp Gly
<210> 10
   <211> 15
   <212> PRT
   <213> Epstein-Barr virus
<400> 10
   Glu His Asp Leu Glu Arg Gly Pro Pro Gly Pro Arg Arg Pro Pro
   1               5                   10                  15
<210> 11
   <211> 16
   <212> PRT
   <213> Epstein-Barr virus
<400> 11
   Ile Ile Phe Ile Phe Arg Arg Asp Leu Leu Cys Pro Leu Gly Ala Leu
   1               5                   10                  15
<210> 12
   <211> 16
   <212> PRT
   <213> Epstein-Barr virus
<400> 12
   Ala Leu Trp Asn Leu His Gly Gln Ala Leu Phe Leu Gly Ile Val Leu
   1 5 10 15
<210> 13
   <211> 17
   <212> PRT
   <213> Epstein-Barr virus
<400> 13
   Ala Ile Leu Ile Trp Met Tyr Tyr His Gly Pro Arg His Ser Asp Glu
   1 5 10 15
   His
<210> 14
   <211> 15
   <212> PRT
   <213> Epstein-Barr virus
<400> 14
   His Asp Pro Leu Pro His Ser Pro Ser Asp Ser Ala Gly Asn Asp
   1 5 10 15
<210> 15
   <211> 27
   <212> PRT
   <213> Epstein-Barr virus
<400> 15
   Ser Gly His Glu Ser Asp Ser Asn Ser Asn Glu Gly Arg His His Leu
   1 5 10 15
   Leu Val Ser Gly Ala Gly Asp Gly Pro Pro Leu
   20 25
<210> 16
   <211> 31
   <212> PRT
   <213> Epstein-Barr virus
<400> 16
   Asp Pro His Leu Pro Thr Leu Leu Leu Gly Thr Ser Gly Ser Gly Gly
   1 5 10 15
   Asp Asp Asp Asp Pro His Gly Pro Val Gln Leu Ser Tyr Tyr Asp
   20 25 30
<210> 17
   <211> 20
   <212> PRT
   <213> Epstein-Barr virus
<400> 17
   Met Gly Ser Leu Glu Met Val Pro Met Gly Ala Gly Pro Pro Ser Pro
   1 5 10 15
   Gly Gly Asp Pro
   20
<210> 18
   <211> 25
   <212> PRT
   <213> Epstein-Barr virus
<400> 18
   Glu Asp Pro Tyr Trp Gly Asn Gly Asp Arg His Ser Asp Tyr Gln Pro
   1 5 10 15
   Leu Gly Thr Gln Asp Gln Ser Leu Tyr
   20 25
<210> 19
   <211> 15
   <212> PRT
   <213> Epstein-Barr virus
<400> 19
   Ala Ser Ser Tyr Ala Ala Ala Gln Arg Lys Leu Leu Thr Pro Val
   1 5 10 15
<210> 20
   <211> 21
   <212> PRT
   <213> Epstein-Barr virus
<400> 20
   Val Leu Val Met Leu Val Leu Leu Ile Leu Ala Tyr Arg Arg Arg Trp
   1 5 10 15
   Arg Arg Leu Thr Val
   20
<210> 21
   <211> 17
   <212> PRT
   <213> Epstein-Barr virus
<400> 21
   Gly Gly Ser Ile Leu Gln Thr Asn Phe Lys Ser Leu Ser Ser Thr Glu
   1 5 10 15
   Phe
<210> 22
   <211> 18
   <212> PRT
   <213> Epstein-Barr virus
<400> 22
   Leu Thr Glu Trp Gly Ser Gly Asn Arg Thr Tyr Gly Pro Val Phe Met
   1 5 10 15
   Cys Leu
<210> 23
   <211> 13
   <212> PRT
   <213> artificial sequence
<220>
   <223> HA 306-318
<400> 23
   Pro Lys Tyr Val Lys Gln Asn Thr Leu Lys Leu Ala Thr
   1 5 10
<210> 24
   <211> 15
   <212> PRT
   <213> artificial sequence
<220>
   <223> A3 152-166
<400> 24
   Glu Ala Glu Gln Leu Arg Ala Tyr Leu Asp Gly Thr Gly Val Glu
   1 5 10 15
<210> 25
   <211> 15
   <212> PRT
   <213> artificial sequence
<220>
   <223> MT 2-16
<400> 25
   Ala Lys Thr Ile Ala Tyr Asp Glu Glu Ala Arg Arg Gly Leu Glu
   1 5 10 15
<210> 26
   <211> 13
   <212> PRT
   <213> artificial sequence
<220>
   <223> YKL
<400> 26
   Ala Ala Tyr Ala Ala Ala Lys Ala Ala Ala Leu Ala Ala
   1 5 10
<210> 27
   <211> 16
   <212> PRT
   <213> artificial sequence
<220>
   <223> B1 21-36
<400> 27
   Thr Glu Arg Val Arg Leu Val Thr Arg His Ile Tyr Asn Arg Glu Glu
   1 5 10 15
<210> 28
   <211> 20
   <212> PRT
   <213> artificial sequence
<220>
<223> LOL 191-210
<400> 28
   Glu Ser Trp Gly Ala Val Trp Arg Ile Asp Thr Pro Asp Lys Leu Thr
   1 5 10 15
   Gly Pro Phe Thr
   20
<210> 29
   <211> 14
   <212> PRT
   <213> artificial sequence
<220>
   <223> E2/E168
<400> 29
   Ala Gly Asp Leu Leu Ala Ile Glu Thr Asp Lys Ala Thr Ile
   1 5 10
<210> 30
   <211> 17
   <212> PRT
   <213> artificial sequence
<220>
   <223> peptide Oxy 271-287
<400> 30

## Description

La présente invention est relative à des peptides immunogènes issus des antigènes de latence de type I et II d'EBV comprenant au moins un épitope T CD4+ apte à être reconnu par la majorité des individus de la population caucasienne, et à leurs applications diagnostiques et thérapeutiques.

Le virus Epstein-Barr (EBV) est un herpes virus oncogène de tropisme lymphocytaire B, infectant 95 % de la population adulte. Après la primo-infection, généralement asymptomatique chez l'enfant et parfois responsable de la mononucléose infectieuse, notamment chez l'adolescent, l'EBV persiste à l'état latent dans les lymphocytes B pendant toute la vie de l'individu. Alors que dans la majorité des cas, l'infection par EBV est contrôlée de manière efficace par le système immunitaire (porteurs sains), dans certains cas, elle est associée à différentes pathologies tumorales que l'on peut distinguer par l'expression des antigènes de latence.

La latence de type 1 correspond à l'expression isolée de l'antigène EBNA1. Ce profil d'expression est retrouvé dans les lymphomes de Burkitt de type endémique rencontrés principalement en Afrique et en Nouvelle Guinée (à la différence de la forme sporadique en général non liée à EBV). Ce type de latence se caractérise par la faible reconnaissance des cellules tumorales par les lymphocytes T CD8+ cytotoxiques. Cette faible capacité de reconnaissance s'explique par : (i) l'absence d'épitopes dérivés d'EBNA1 présentés au niveau du CMH de classe 1 (répétitions de séquences Glycine-Alanine empêchant la dégradation par le protéasome); (ii) la diminution des molécules d'adhésion ; (iii) la diminution d'expression des proteines TAP 1 et 2 ; (iv) la faible expression des molécules HLA de classe 1 (ou HLA 1). Les cellules tumorales peuvent néanmoins être reconnues par des lymphocytes T CD4+ cytotoxiques puisqu'elles présentent une expression normale des molécules HLA de classe II (ou HLA 11), associée à un processing normal.

Dans la latence de type II, on retrouve une expression des antigènes de latence limitée à EBNA1, LMP1 et LMP2. Ce type de latence est rencontré dans :
- le carcinome indifferencié du naso-pharynx (NPC) observé
principalement en Chine du Sud et en Afrique du Nord.
- 40 a 50% des maladies de Hodgkin. L'association avec EBV est plus fréquente dans les formes à cellularité mixte (50-75%) que dans les formes scléro-nodulaires (15-30%).
- certains lymphomes T et NK notamment les lymphomes T/NK naso-sinusiens.

En ce qui concerne la maladie de Hodgkin, on note une expression normale des molécules HLA de classe I et des transporteurs TAP 1 et 2, ainsi qu'une expression importante des molécules HLA de classe Il par les cellules de Reed-Steinberg. Toutefois, la latence de type II se caractérise par l'absence d'épitopes immunodominants (EBNA 3A, 3B, 3C). En effet, s'il existe une réponse CTL dirigée contre les protéines LMP1 et LMP2, cette dernière reste faible. Il paraît donc intéressant de stimuler cette réponse dans le cadre de protocoles d'immunothérapie.

La latence de type III qui correspond à l'expression de l'ensemble des protéines de latence (EBNA1, EBNA2, EBNA3A-C, EBNA-LP, LMP1 et LMP2), s'observe dans les lymphomes post-transplantation, liés à un état d'immunodépression sévère.

Le contrôle de l'infection latente à EBV par des lymphocytes T spécifiques d'EBV est primordial (Rooney et al., Lancet, 1995, 345, 913; Munz, J. Exp. Med., 2000, 191, 1649-1660; Leen et al. J. Virol., 2001, 75, 8649-8659). Il est destiné à empêcher une prolifération non contrôlée de cellules infectées par EBV que l'on observe chez les sujets immunodéprimés (syndrômes prolifératifs post-transplantation, latence de type III).

De manière schématique, les protéines EBNA3A-C et LMP2 sont les principales cibles des lymphocytes T CD8+ alors que EBNA1 et LMP1 sont principalement reconnues -voire presque exclusivement pour EBNA1- par des lymphocytes T CD4+. Par conséquent, le contrôle immunitaire des tumeurs associées aux latences de type 1 et Il d'EBV repose en grande partie sur les lymphocytes T CD4+ cytotoxiques capables de reconnaître et de lyser les cellules cibles exprimant des épitopes d'EBNA1 et/ou de LMP1, présentés par des molécules HLA II. D'autre part, les lymphocytes T CD4+ sont nécessaires au maintien *in vivo* d'une réponse cytotoxique médiée par des lymphocytes TCD8+, capables de reconnaître et de lyser les cellules cibles exprimant des épitopes de LMP2.

Il n'existe pas actuellement de protocole d'immunothérapie des pathologies tumorales associées à une latence de type I ou II, qui soit réellement efficace et facile à mettre en oeuvre. En effet, l'immunothérapie cellulaire qui repose sur la préparation de lymphoytes T cytotoxiques polyclonaux obtenus par stimulation *in vitro* de lymphocytes T avec des lignées lymphoblastoïdes B autologues, transformées par EBV (LCL) est longue à mettre en oeuvre (obtention de LCL en 4 à 6 semaines) et peu efficace dans la mesure où le taux d'expansion des lymphocytes T spécifiques d'EBV est faible et nécessite l'ajout d'un cocktail mitogène chez les patients atteints de maladie de Hodgkin ; en outre, aucune rémission complète des tumeurs n'est observée.

Ces observations sont en faveur de l'utilisation de peptides antigéniques spécifiques des antigènes de latence EBNA1, LMP1 et LMP2 d'EBV et capables de stimuler une forte réponse T CD4+, en immunothérapie (vaccination ou thérapie cellulaire), pour la prévention et le traitement des pathologies tumorales associées aux latences de type 1 ou de type II.

De tels peptides qui sont reconnus par des cellules T CD4+ spécifiques d'EBV sont également utiles comme réactifs dans un test de diagnostic de l'infection par EBV ou de pathologies tumorales associées, ou bien de suivi du traitement chez l'Homme, reposant sur la détection directe (test de prolifération lymphocytaire) ou indirecte (production d'anticorps, de cytokines...) desdites cellules T CD4+.

L'activation des lymphocytes T CD4+ ou cellules T CD4+ se fait sous l'effet de la présentation de peptides antigéniques par les molécules du complexe majeur d'histocompatibilité de type II que portent les cellules présentatrices d'antigène ; chez l'homme, elles sont dénommées molécules HLA II pour *Human Leucocyte Antigen type II.* Ces peptides antigéniques, appelés épitopes T, résultent de la dégradation protéolytique des antigènes par les cellules présentatrices d'antigène. Ils ont des longueurs variables, généralement de 13 à 25 acides aminés et possèdent une séquence qui les rend capables de se lier aux molécules HLA II. II est bien connu qu'au même titre que l'antigène natif, un peptide comprenant un épitope T CD4+ est capable de stimuler *in vitro* des cellules T CD4+ qui lui sont spécifiques ou de les recruter *in vivo.* II est donc suffisant pour induire une réponse T CD4+.

Toutefois, un des problèmes majeurs qui limite l'utilisation de ces peptides comme antigène est l'identification des épitopes T CD4+, étant donné que leur séquence varie d'un individu à l'autre en raison du polymorphisme des molécules HLA II. En effet, les molécules HLA II sont des hétérodimères constitués d'une chaîne alpha (α) et d'une chaîne béta (β) polymorphes. II existe quatre types de molécules HLA II par individu (2 HLA-DR, 1 HLA-DQ et 1 HLA-DP), dénommées en fonction de l'allèle codant la chaîne béta qui est la plus polymorphe. La molécule HLA-DR est très polymorphe. En effet, alors que sa chaîne alpha ne compte que 3 allèles, la chaîne béta (β) codée par le gène DRB1, qui est la plus exprimée, compte à ce jour 458 allèles. Pour les molécules HLA-DQ et HLA-DP, les deux chaînes (α et β) qui les constituent sont polymorphes mais elles présentent moins d'allèles. On a dénombré 8 allèles DQA1 (chaîne α de HLA-DQ), 56 allèles DQB1 (chaîne β de HLA-DQ), 20 allèles DPA1 (chaîne α de HLA-DP) et 110 allèles DPB1 (chaîne β de HLA-DP). Cependant, la combinaison entre les deux chaînes α et β codées par ces allèles donne naissance à de nombreuses molécules HLA-DQ et HLA-DP. Du fait de ce polymorphisme, ces isoformes possèdent des propriétés de liaison différentes entre elles, ce qui implique qu'elles peuvent lier des peptides différents d'un même antigène. Ainsi, chaque individu reconnaît dans un antigène un ensemble de peptides dont la nature dépend des molécules HLA II qui le caractérisent. Comme il existe un grand nombre d'allèles HLA II, il existe donc dans une séquence donnée un répertoire important d'épitopes T de séquences très différentes, chacun spécifique d'un allèle différent.

Ainsi, un peptide capable de stimuler une réponse T CD4+ spécifique d'EBV chez quelques individus peut être inactif chez la majorité des autres individus, parce que ces derniers ne reconnaissent pas les antigènes d'EBV par les mêmes épitopes.

La plupart des épitopes T CD4+ des antigènes EBNA1, LMP1 et LMP2 connus, possèdent une restriction définie pour une seule molécule HLA DR, DQ ou DP (Tableau 1).

### DESCRIPTION MODIFIEE

**Tableau I : Epitopes T CD4+ des antigènes EBNA1, LMP1 et LMP2**

| **Antigène d'EBV** | **Epitope T CD4+** | **Restriction HLA II** | **Référence** |
|---|---|---|---|
| **EBNA1** | **475-489** | DR11 | Leen et al. J. Virol., 2001, 75, 8649-8659 |
| | **475-489** | DP | Taylor et al., J. Virol., 2004,78, 768-778 |
| | **481-500** | DQ | Paludan et al., J. Immunol., 2002, 169, 1593-1603 |
| | **482-496** | DR4, DR7 DR11, DR15 | Kruger et al., J. Immunother., 2003, 26, 212-221 |
| | **485-499** | [DR11,DR13,DR15, DQ1,DQ3,DQ6]* | Leen et al., précité |
| | **514-527** | DR1 | Paludan et al., précité |
| | **514-533** | DR1* | Leen et al., précité |
| | **515-527** | DR1 | Khanna et al., Virology, 1995, 214, 633-637 |
| | **515-528** | DR1 | Leen et al., précité Taylor et al., précité |
| | **518-530** | DP3/DR4 | Voo et al., Cancer Res., 2002, 62, 7195-7199 & Demande US 2004/0141995 |
| | **519-533** | DR4,DQ3* | Leen et al., précité |
| | **529-543** | DR14 | Taylor et al., précité |
| | **529-543** | [DR13, DQ3, DQ5,DQ6]* | Leen et al., précité |
| **LMP1** | **66-85**** | Non-déterminée | Demande WO 2004/001424 |
| | 130-144 | [DR1,DR4,DQ1,DQ3]* | Leen et al., précité |
| | 208-224 | Non-déterminée | Demande WO 2004/041849 |
| | 212-226 | DQ2* | Leen et al., précité |
| | 340-354 | Non-déterminée | Leen et al., précité |
| **LMP2** | 149-163 | [DR1,DR13,DQ5]* | Leen et al., précité |
| | 169-182 | [DR1,DR13,DQ5,DQ6]* | Leen et al., précité |
| | **224-243** | Non-déterminée | Leen et al., précité |
| | **369-388*** | Non-déterminée | Demande WO 2004/01424 |
| | 385-398 | Non-déterminée | Leen et al., précité |

| | | | |
|---|---|---|---|
| * élément de restriction potentiel déterminé en fonction de l'haplotype HLA II des individus répondeurs. ** utilisation pour induire une tolérance immunitaire dans le but de traiter des maladies autoimmunes. | | | |

D'autres dérivés du virus Epstein-Barr ont été décrits en J. Biol. Chem 270(35), pages 20556-20559 (1995) et dans WO-A-2 0040 001 424.

En conséquence, de tels peptides ne seront généralement pas reconnus par l'ensemble des molécules HLA II prépondérantes dans la population caucasienne. En effet, pour les molécules HLA-DR, par exemple, une dizaine d'allèles sont nécessaires pour couvrir plus de 60 % de la fréquence génique retrouvée dans la population caucasienne et donc concerner plus de 85 % de cette population.

Les inventeurs ont identifié des épitopes T CD4+ dérivés des antigènes de latence EBNA1, LMP1 et LMP2 d'EBV, lesquels épitopes sont aptes à être reconnus par les molécules HLA II prépondérantes dans la population caucasienne et donc capables d'induire une réponse T CD4+ spécifique d'EBV chez la majorité des individus de cette population recevant une vaccination ou une thérapie cellulaire à l'aide de peptides incluant ces épitopes.

La présente invention a en conséquence pour objet, un peptide immunogène du virus Epstein-Barr (EBV) comprenant au moins un épitope T CD4+ de l'un des antigènes de latence EBNA1, LMP1 ou LMP2, apte à être présenté par au moins 7 molécules HLA II différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, lequel peptide est sélectionné dans le groupe constitué par :
a) les peptides de respectivement 26, 26 et 24 acides aminés dont la séquence s'étend des positions 475 à 500, 514 à 539, et 529 à 552 d'EBNA1,
b) le peptide de 16 acides aminés dont la séquence s'étend des positions 68 à 83 de LMP1, et
c) les peptides de respectivement 21 et 17 acides aminés dont la séquence s'étend des positions 224 à 244 et 372 à 388 de LMP2.

Les peptides selon l'invention dont les séquences sont celles précisées ci-dessus présentent des propriétés distinctes des peptides de l'art antérieur et notamment les propriétés suivantes :
- activité de liaison aux molécules HLA II : les peptides selon l'invention possèdent une forte affinité pour la majorité des molécules HLA II prépondérantes dans la population caucasienne et sont donc aptes à être présentés par des molécules HLA II chez la majorité des individus de la population caucasienne. Ces peptides possèdent une activité de liaison < 1000 nM pour au moins 7 des
molécules HLA II choisies parmi HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4 qui correspondent aux molécules HLA II dont la fréquence allélique est supérieure à 5 % dans la population caucasienne. L'activité de liaison peut-être mesurée par un test de liaison HLA II/peptide, en compétition, avec une révélation immuno-enzymatique, selon le principe décrit dans le Brevet US G,649,169 pour les molécules HLA-DR et dans la Demande Internationale PCT WO 03/040299 pour les molécules HLA-DP4, ainsi que dans les articles aux noms de Texier et al., J. Immunol., 2000, 164, 3177 et Eur. J. Immunol., 2001, 31, 1837 et Castelli et al., J. Immunol, 2002, 169, 6928-6934.
- immunogénicité : les peptides selon l'invention sont reconnus par des lymphocytes T CD4+ chez la majorité des individus de la population caucasienne et contiennent des épitopes T naturellement présentés au cours de l'infection par EBV. En conséquence, ces peptides sont donc capables d'induire des cellules T CD4+ spécifiques d'EBV à partir des précurseurs présents chez la majorité des individus naïfs ou bien de stimuler de telles cellules chez la majorité des individus séropositifs pour EBV. L'immunogénicité des peptides peut être déterminée, notamment à partir de cellules mononucléées du sang périphérique (PBMCs), par tout essai approprié connu de l'Homme du métier comme par exemple : un test de prolifération cellulaire, un test ELISPOT (dosage des cellules productrices de cytokines) ou un test de dosage de cytokines (IFN-γ, IL-2, IL-4 et IL-10).
- activité cytotoxique : des clones de lymphocytes T CD4+ de type Th1 sont isolés à partir des lymphocytes T générés par stimulation *in vitro* de PBMC, à l'aide des peptides selon l'invention. Les clones ainsi obtenus sont mis en présence de cibles EBV positives de même spécificité HLA : lymphocytes issus du même individu et transformés par EBV (LCL) ou des lignées tumorales (Hodgkin, lymphomes T/NK) EBV positives en cas d'identité HLA. La présence de lymphocytes T CD4+ cytotoxiques spécifiques des antigènes de latence de type 1 ou II d'EBV, capables de lyser les cellules cibles EBV positives de même spécificité, est analysée par un test de cytotoxicité classique, notamment par mesure du relargage du ⁵¹Cr.

L'invention englobe les peptides dont la séquence correspond à celle d'un fragment de la protéine EBNA1, LMP1 ou LMP2 de n'importe quel isolat d'EBV, incluant les variants naturels isolés à partir d'individus infectés par EBV. Les séquences du génome et des protéines correspondantes des différents isolats d'EBV sont disponibles dans les banques de données, notamment dans celle du NCBI (http://www.ncbi.nlm.nih.gov).

Les positions des peptides sont indiquées relativement aux séquences présentant les numéros d'accès suivants dans la base de données NCB] : NP_039875 (EBNA1), CAA26023 (LMP1) et AAA45887 (LMP2). Par exemple le peptide de 26 acides aminés qui s'étend des positions 475 à 500 d'EBNA1, s'étend du résidu d'asparagine (N) en position 475 au résidu d'acide glutamique (E) en position 500 de la séquence NCBI NP_039875 ; il est notamment représenté par la séquence NPKFENIAEGLRALLARSHVERTTDE (SEQ ID NO : 4).

A partir de ces indications, l'Homme du métier est en mesure de déterminer facilement les positions correspondantes dans les protéines d'autres isolats d'EBV, qui peuvent varier de quelques acides aminés du fait de courtes insertions et/ou délétions dans la séquence en acides aminés desdites protéines.

Selon un mode de réalisation avantageux dudit peptide, ladite molécule HLA II comprend une chaîne β codée respectivement par les allèles DRB1*0101 (molécule HLA-DR1), DRB1*0301 (molécule HLA-DR3) DRB1*0401 (molécule HLA-DR4) DRB1*0701 (molécule HLA-DR7), DRB1*1101 (molécule HLA-DR11), DRB1*1301 (molécule HLA-DR13), DRB1 *1501 (molécule HLA-DR15), DRB3*0101 (molécule HLA-DRB3), DRB5*0101 (molécule HLA-DRB5), DP*0401 ou DP*0402 (molécule HLA-DP4).

Selon une disposition avantageuse de ce mode de réalisation, ledit peptide est sélectionné dans le groupe constitué par les séquences SEQ ID NO :4, 5, 6, 11, 20 et 21.

La présente invention a également pour objet un dérivé polyépitopique, caractérisé en ce qu'il comprend au moins deux épitopes identiques ou différents d'EBV, incluant au moins un épitope T CD4+ dont la séquence est celle d'un peptide tel que défini ci-dessus.

Au sens de la présente invention on entend par dérivé polyépitopique, une séquence artificielle ou synthétique, obtenue à partir de fragments d'un ou plusieurs antigènes d'EBV, laquelle séquence ne correspond à aucune séquence naturellement présente dans un antigène d'EBV.

De préférence, ledit dérivé polyépitopique présente une longueur de 20 à 1000 acides aminés, de préférence de 20 à 100 acides aminés.

Ledit dérivé polyépitopique comprend avantageusement une étiquette fusionnée à l'une de ses extrémités, pour la purification ou la détection dudit fragment. L'étiquette, notamment une séquence polyhistidine ou un épitope B d'un autre virus, est de préférence séparée de la séquence polyépitopique par un site de clivage d'une protéase de façon à isoler la séquence polyépitopique, à partir de 1a fusion.

Selon un mode de réalisation avantageux dudit dérivé polyépitopique, il comprend un épitope T CD4+ dont la séquence est celle d'un peptide tel que défini ci-dessus et au moins un autre épitope sélectionné dans le groupe constitué par :
- un épitope T CD8+ d'une protéine d'EBV présenté par une molécules HLA 1 et reconnu par des lymphocytes T cytotoxiques spécifiques d'EBV ; les séquences des épitopes T CD8+ d'EBV sont connus de l'Homme du métier et incluent notamment les peptides 51-60, 125-133, 156-164 et 159-167 de LMP1 et les peptides 131-139, 200-208, 236-244, 340-350, 419-427, 426-434, 442-451, 447-455 et 453-461 de LMP2.
- un épitope B d'un antigène d'EBV reconnu spécifiquement par des anticorps dirigés contre EBV, et
- un épitope T CD4+ universel naturel ou synthétique tel que le peptide de la toxine tétanique TT 830-846 (O'SULLIVAN et al., J. Immuno1., 1991, 147, 2663-2669), le peptide de l'hémagglutinine du virus de la grippe HA 307-3l9 (O'SULLIVAN et al, précité), le peptide PADRE (KXVAAWTLKAA; Alexander et al., Immunity, 1994, 1, 751-761), le peptide 45-69 de la protéine Nef du VIH-1 et des peptides issus des antigènes de *Plasmodium falciparum* tels que le peptide CS.T3
(SINIGAGLIA et al., Nature, 1988, 336, 778-780) et les peptides CSP, SSP2, LSA-1 et EXP-1 (DOOLAN et al., J. Immunol., 2000,165, 1123-1137).

La combinaison de l'épitope T CD4+ avec l'un au moins des épitopes tels que définis ci-dessus permet avantageusement de déclencher ou de moduler une réponse immunitaire anti-EBV.

La présente invention a également pour objet un lipopeptide, **caractérisé en ce qu**'il comprend un peptide ou un dérivé polyépitopique tels que définis ci-dessus. Ledit lipopeptide est notamment obtenu par addition d'un lipide sur une fonction α-aminée ou sur une fonction réactive de la chaîne latérale d'un acide aminé dudit peptide ou dérivé polyépitopique ; il peut comprendre une ou plusieurs chaînes dérivées d'acides gras en C₄₋₂₀, éventuellement ramifiées ou insaturées (acide palmitique, acide oléique, acide linoléique, acide linolénique, acide 2-amino hexadécanoïque, pimélautide, trimétauxide) ou un dérivé d'un stéroïde. La partie lipidique préférée est notamment représentée par un groupe N^{α}-acétyl-lysine N^{ε}(palmitoyl), également dénommé Ac-K(Pam).

La présente invention a également pour objet une protéine de fusion, **caractérisée en ce qu**'elle est constituée par une protéine ou un fragment de protéine hétérologue, fusionnés avec un peptide ou un dérivé polyépitopique tels que définis ci-dessus. Le peptide ou le dérivé polyépitopique peuvent être fusionnés avec l'extrémité NH₂ ou COOH de ladite protéine ou insérés dans la séquence de ladite protéine.

Au sens de la présente invention, on entend par protéine hétérologue, relativement aux peptides dérivés respectivement d'EBNA1, LMP1 et LMP2, des protéines autres que EBNA1, LMP1 et LMP2.

Avantageusement ladite protéine de fusion est constituée par un peptide d'EBV tel que défini ci-dessus, fusionné avec l'une des chaînes d'une molécule HLA II choisie parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, de préférence la chaîne béta, ou bien avec un fragment de celle-ci correspondant à une molécule HLA II soluble, notamment un fragment correspondant au domaine extracellulaire précédé du peptide signal homologue ou d'un peptide signal hétérologue. Ledit peptide d'EBV est avantageusement inséré entre le peptide signal et l'extrémité NH₂ du domaine extracellulaire de la chaîne β, comme décrit pour la molécule HLA-DR ( Kolzin et al., PNAS, 2000, 97, 291-296).

Alternativement, ledit peptide d'EBV ou ledit dérivé polyépitopique sont fusionnés avec une protéine facilitant leur purification ou leur détection, connue de l'Homme du métier comme notamment la glutathione-S-transférase (GST) et les protéines fluorescentes (GFP et dérivées). Dans ce cas, la séquence du peptide ou du dérivé polyépitopique d'intérêt est de préférence séparée du reste de la protéine par un site de clivage d'une protéase, pour faciliter la purification dudit peptide ou dudit fragment polyépitopique.

La présente invention a également pour objet un polynucléotide, **caractérisé en ce qu**'il code pour un peptide, un dérivé polyépitopique ou une protéine de fusion tels que définis ci-dessus.

Conformément à l'invention, la séquence dudit polynucléotide est celle de l'ADNc codant ledit peptide ou dérivé polyépitopique ou ladite protéine de fusion. Ladite séquence peut avantageusement être modifiée de façon à ce que l'usage des codons soit optimal chez l'hôte dans lequel elle est exprimée.

L'objet de l'invention englobe également les polynucléotides recombinants comprenant au moins un polynucléotide conforme à l'invention lié à au moins une séquence hétérologue.

Au sens de 1a présente invention, on entend par séquence hétérologue relativement à une séquence d'acide nucléique codant un peptide d'EBV, toute séquence d'acide nucléique autre que celles qui, dans 1a nature, sont immédiatement adjacentes à ladite séquence d'acide nucléique codant ledit peptide d'EBV.

L'objet de la présente invention englobe en particulier :
a) des cassettes d'expression comprenant au moins un polynucléotide tel que défini ci-dessus, sous le contrôle de séquences régulatrices de la transcription et éventuellement de la traduction appropriées (promoteur, activateur, intron, codon d'initiation (ATG), codon stop, signal de polyadénylation), et
b) des vecteurs recombinants comprenant un polynucléotide conforme à l'invention. Avantageusement ces vecteurs sont des vecteurs d'expression comprenant au moins une cassette d'expression telle que définie ci-dessus.

La présente invention a en outre pour objet des cellules hôtes procaryotes ou eucaryotes modifiées par au moins un polynucléotide ou un vecteur tel que défini ci-dessus.

De nombreux vecteurs dans lesquels on peut insérer une molécule d'acide nucléique d'intérêt afin de l'introduire et de la maintenir dans une cellule hôte eucaryote ou procaryote, sont connus en eux-mêmes ; le choix d'un vecteur approprié dépend de l'utilisation envisagée pour ce vecteur (par exemple réplication de la séquence d'intérêt, expression de cette séquence, maintien de cette séquence sous forme extrachromosomique, ou bien intégration dans le matériel chromosomique de l'hôte), ainsi que de la nature de la cellule hôte. Par exemple, on peut utiliser entre autres des vecteurs viraux tels que les adénovirus, les rétrovirus, les lentivirus, les AAV et les baculovirus, dans lesquels a été insérée préalablement la séquence d'intérêt ; on peut également associer ladite séquence (isolée ou insérée dans un vecteur plasmidique) avec une substance lui permettant de franchir la membrane des cellules hôtes, telle qu'un transporteur comme un nanotransporteur ou une préparation de liposomes, ou de polymères cationiques, ou bien l'introduire dans ladite cellule hôte en utilisant des méthodes physiques telles que l'électroporation ou la microinjection. En outre, on peut avantageusement combiner ces méthodes, par exemple en utilisant l'électroporation associée à des liposomes.

Les polynucléotides, les vecteurs recombinants et les cellules transformées tels que définis ci-dessus, sont utiles notamment pour la production des peptides, fragments polyépitopiques et protéines de fusion selon l'invention.

Les polynucléotides selon l'invention sont obtenus par les méthodes classiques, connues en elles-mêmes, en suivant les protocoles standards tels que ceux décrits dans Current Protocols in Molecular Biology (Frederick M. A USUBEL, 2000, Wiley and son Inc, Library of Congress, USA*).* Par exemple, ils peuvent être obtenus par amplification d'une séquence nucléique par PCR ou RT-PCR, par criblage de banques d'ADN génomique par hybridation avec une sonde homologue, ou bien par synthèse chimique totale ou partielle. Les vecteurs recombinants sont construits et introduits dans des cellules hôtes par les méthodes classiques d'ADN recombinant et de génie génétique, qui sont connues en elles-mêmes.

Les peptides et leurs dérivés (variants, peptides modifiés, lipopeptides, dérivés polyépitopiques, protéines de fusion) tels que définis ci-dessus, sont préparés par les techniques classiques connues de l'Homme du métier, notamment par synthèse en phase solide ou liquide ou par expression d'un ADN recombinant dans un système cellulaire approprié (eucaryote ou procaryote).

De manière plus précise,
- les peptides et leurs dérivés (variants, fragments polyépitopiques) peuvent être synthétisés en phase solide, selon la technique Fmoc, originellement décrite par Merrifield et al. (J. Am. Chem. Soc., 1964, 85: 2149-) et purifiés par chromatographie liquide haute performance en phase inverse,
- les lipopeptides peuvent être notamment préparés selon le procédé décrit dans les Demandes Internationales WO 99/40113 ou WO 99/51630.
- les peptides et dérivés tels que les variants, les dérivés polyépitopiques et les protéines de fusion peuvent également être produits à partir des ADNc correspondants, obtenus par tout moyen connu de l'homme du métier ; l'ADNc est cloné dans un vecteur d'expression eucaryote ou procaryote et la protéine ou le fragment produits dans les cellules modifiées par le vecteur recombinant sont purifiés par tout moyen approprié, notamment par chromatographie d'affinité.

La présente invention a également pour objet une composition immunogène ou vaccinale, caractérisée en ce qu'elle comprend au moins un peptide d'EBV, un dérivé polyépitopique, un lipopeptide ou un vecteur tels que définis ci-dessus, et un véhicule pharmaceutiquement acceptable, et/ou une substance porteuse, et/ou un adjuvant.

La composition immunogène selon l'invention se présente sous une forme galénique adaptée à une administration par voie parentérale (sous-cutanée, intramusculaire, intraveineuse), entérale (orale, sublinguale), ou locale (rectale, vaginale).

Les véhicules pharmaceutiquement acceptables, les substances porteuses et les adjuvants sont ceux classiquement utilisés.

Les adjuvants sont avantageusement choisis dans le groupe constitué par : des émulsions huileuses, des substances minérales, des extraits bactériens, la saponine, l'hydroxyde d'alumine, le monophosphoryl -lipide A et le squalène.

Les substances porteuses sont avantageusement sélectionnées dans le groupe constitué par : les liposomes unilamellaires ou multilamellaires, les ISCOMS, les virosomes, les pseudoparticules virales, les micelles de saponine, les microsphères solides de nature saccharidique (poly(lactide-co-glycolide)) ou aurifère, et les nanoparticules.

De préférence, ladite composition immunogène comprend au moins deux des six peptides d'EBV incluant un épitope T CD4+ tels que définis ci-dessus, de préférence trois à cinq peptides, de manière préférée les six peptides, sous forme d'un mélange de peptides ou de lipopeptides, d'un dérivé polyépitopique ou d'un vecteur d'expression codant lesdits peptides ou ledit fragment.

De manière préférée, ladite composition comprend également, au moins un autre peptide incluant un épitope sélectionné dans le groupe constitué par : un épitope T CD4+ universel ou un épitope T CD8+ d'EBV, tels que définis ci-dessus.

La présente invention a également pour objet un peptide, un dérivé polyépitopique, un lipopeptide ou un vecteur tels que défini ci-dessus comme vaccin pour la prévention et/ou le traitement d'une infection par EBV ou d'une pathologie tumorale associée.

La présente invention a également pour objet l'utilisation d'un peptide, d'un dérivé polyépitopique, d'un lipopeptide ou d'un vecteur tels que défini ci-dessus, pour la préparation d'un vaccin destiné à la prévention et/ou au traitement d'une infection par EBV ou d'une pathologie tumorale associée.

Les peptides selon la présente invention et les produits dérivés (dérivé polyépitopique, lipopeptide, vecteur recombinant) peuvent être utilisés en immunothérapie dans le traitement des tumeurs associées à EBV et notamment celles présentant une latence de type I ou II. Lesdits peptides ou produits dérivés sont utilisés, soit comme vaccin, soit en thérapie cellulaire, ou bien encore par une combinaison des deux approches.

La thérapie cellulaire comprend, la préparation de lymphocytes T CD4+ spécifiques d'EBV par un protocole classique de stimulation *in vitro* comprenant l'isolement de cellules mononucléées du sang périphérique (PBMC) d'un patient à traiter ou d'un donneur volontaire de phénotype HLA identique ou partiellement identique, et la mise en culture des PBMC en présence de peptide(s), de façon à induire la prolifération de lymphocytes T CD4+ spécifiques d'EBV. Dans une seconde étape les lymphocytes T CD4+ spécifiques d'EBV sont réinjectés au patient.

La présente invention a également pour objet un réactif de diagnostic et de suivi de l'évolution d'une infection par EBV ou d'une pathologie tumorale associée, caractérisé en ce qu'il comprend au moins un peptide tel que défini ci-dessus, éventuellement marqué ou complexé, notamment complexé à des molécules HLA II marquées, sous la forme de complexes multimériques comme des tétramères.

La présente invention a également pour objet l'utilisation d'un peptide tel que défini ci-dessus, pour la préparation d'un réactif de diagnostic et de suivi de l'évolution d'une infection par EBV ou d'une pathologie tumorale associée.

Au sens de la présente invention, on entend par diagnostic ou suivi de l'évolution d'une infection par EBV ou d'une pathologie tumorale associée, l'évaluation de la réponse immunitaire T CD4+ spécifique d'EBV au cours d'une infection par EBV, d'une pathologie associée à EBV, ou bien d'un protocole d'immunothérapie ou de vaccination anti-EBV. La détection est effectuée à partir d'un échantillon biologique contenant des cellules T CD4+, notamment un échantillon de cellules mononucléées isolées à partir d'un prélèvement de sang périphérique (PBMCs).

La présente invention a également pour objet une méthode de diagnostic et de suivi de l'évolution d'une infection par EBV ou d'une pathologie tumorale associée, caractérisée en ce qu'elle comprend :
- la mise en contact d'un échantillon biologique dudit individu avec un réactif de diagnostic tel que défini ci-dessus, et
- la détection de lymphocytes T CD4+ spécifiques d'un des antigènes de latence EBNA1, LMP1 ou LMP2 d'EBV, par tout moyen approprié.

La présente invention a également pour objet un kit de détection et de suivi de l'évolution d'une infection par EBV ou d'une pathologie tumorale associée, caractérisé en ce qu'il comprend au moins un réactif tel que défini ci-dessus, associé à un moyen de détection de lymphocytes T CD4+ spécifiques d'un des antigènes de latence EBNA1, LMP1 et LMP2 d'EBV.

La détection des lymphocytes T CD4+ spécifiques d'un antigène de latence, est effectuée par tous moyens, connus en eux-mêmes. Par exemple, on peut utiliser des moyens directs comme les tests de prolifération lymphocytaire ou la cytométrie de flux en présence de complexes multimériques tels que définis ci-dessus, ou bien des moyens indirects comme le dosages de cytokines telles que l'IL-2, l'IL-4, l'IL-5, IL-10 et l'IFN-γ, notamment par des techniques immunoenzymatiques (ELISA, RIA, ELISPOT) ou par cytométrie de flux (dosage des cytokines intracellulaires).

De manière plus précise :

Une suspension de cellules (PBMC, PBMC déplétées en cellules CD8+, lymphocytes T préalablement enrichis par une étape de culture *in vitro* avec les peptides tels que définis ci-dessus ou des lymphocytes T clonés) est cultivée pendant 3 à 5 jours en présence desdits peptides et au besoin de cellules présentatrices appropriées, telles que des cellules dendritiques, des PBMC autologues ou hétérologues, des cellules lymphoblastoïdes telles que celles obtenues après infection par le virus EBV ou des cellules génétiquement modifiées. La présence de cellules T CD4+ spécifiques d'un antigène de latence d'EBV dans la suspension initiale est détectée à l'aide des peptides d'EBV, selon l'une des méthodes suivantes :

### * test de prolifération :

La prolifération des cellules T CD4+ spécifiques d'un antigène de latence d'EBV est mesurée par incorporation de thymidine tritiée dans l'ADN des cellules.

### * test ELISPOT :

Le test ELISPOT permet de révéler la présence de cellules T sécrétant de cytokines (IL-2, IL-4, IL-5, IL-10 et l'IFN-γ), spécifiques d'un peptide tel que défini ci-dessus. Le principe de ce test est décrit dans Czerkinsky et al., J. Immunol. Methods, 1983, 65, 109-121 et Schmittel et al., J. Immunol. Methods, 1997, 210, 167-174, et sa mise en oeuvre est illustrée dans la Demande Internationale WO 99/51630 ou Gahéry-Ségard et al., J. Virol., 2000, 74, 1694-1703.

### * détection des cytokines :

La présence de cellules T spécifiques d'un antigène de latence d'EBV, sécrétant des cytokines telles que l'IL-2, I'IL-4, l'IL-5, IL-10 et l'IFN-γ est détectée, soit par dosage des cytokines présentes dans le surnageant de culture, par un test immunoenzymatique, notamment à l'aide d'un kit commercial, soit par détection des cytokines intracellulaires en cytométrie de flux. Le principe de détection des cytokines intracellulaires est décrit dans Goulder et al. , J. Exp. Med., 2000, 192, 1819-1832 et Maecker et al., J. Immunol. Methods, 2001, 255, 27-40 et sa mise en oeuvre est illustrée dans Draenert et al., J. Immunol. Methods, 2003, 275, 19-29.

### * complexes multimériques

- un échantillon biologique, de préférence des cellules mononucléées du sang périphérique (PBMC), est mis en contact avec des complexes multimériques marqués, notamment par un fluorochrome, formés par la liaison entre des molécules HLA II solubles et des peptides d'EBV tels que définis ci-dessus, et
- les cellules marquées par lesdits complexes multimériques sont analysées, notamment par cytométrie de flux.

De manière avantageuse, préalablement à la mise en contact de l'échantillon biologique avec lesdits complexes, on l'enrichit en cellules T CD4+, en le mettant en contact avec des anticorps anti-CD4 ou par tri indirect, pour éviter une activation non-spécifique.

Les complexes multimériques HLA II/peptide peuvent être préparés à partir de molécules natives extraites de cellules exprimant HLA II ou de molécules recombinantes produites dans des cellules hôte appropriées comme précisé, par exemple dans NOVAK et al. (J. Clin. Investig., 1999, 104, R63-R67) ou dans KURODA et al. (J. Virol., 2000, 74, 18, 8751-8756). Ces molécules HLA II peuvent notamment être tronquées (délétion du domaine transmembranaire) et leur séquence peut être modifiée afin de les rendre solubles ou bien de faciliter l'appariement des chaînes alpha et béta (Novak et al., précité).

Le chargement de molécules HLA II avec le peptide peut se faire par mise en contact d'une préparation de molécules HLA II comme ci-dessus, avec le peptide. Par exemple, des molécules HLA II solubles et biotinylées sont incubées, pendant 72 heures à 37°C, avec un excès d'un facteur 10 de peptides d'EBV tels que définis ci-dessus, dans un tampon phosphate-citrate 10 mM, NaCl 0,15 M à un pH compris entre 4,5 et 7.

Alternativement, la séquence du peptide peut être introduite dans l'une des chaînes de la molécule HLA II sous forme d'une protéine de fusion qui permet la préparation de complexes multimériques HLA II/peptides à partir de cellules hôtes appropriées exprimant ladite protéine de fusion. Lesdits complexes peuvent ensuite être marqués, notamment par la biotine.

Les complexes multimériques de type tétramère sont notamment obtenus en ajoutant aux molécules HLA II chargées, de la streptavidine marquée par un fluorochrome en quantité quatre fois moindre (mole à mole) par rapport aux molécules HLA II, l'ensemble étant ensuite incubé pendant une durée suffisante, par exemple une nuit à température ambiante.

Les complexes multimériques peuvent également être formés, soit par incubation de monomères HLA II/peptides avec des billes magnétiques couplées à la streptavidine comme décrit pour les molécules HLA 1 (Bodinier et al., Nature, 2000, 6, 707-710), soit par insertion de monomères HLA II/peptides dans des vésicules lipidiques comme décrit pour les molécules du CMH de classe II murines (Prakken, Nature Medicine, 2000,6, 1406-1410).

Pour utiliser ces complexes multimériques HLA II/peptide notamment de type tétramère, on met en contact une suspension de cellules (PBMC, PBMC déplétées en cellules CD8+, lymphocytes T préalablement enrichis par une étape de culture *in vitro* avec des peptides d'EBV tels que définis ci-dessus ou des lymphocytes T clonés) avec des complexes multimériques HLA 11/peptide à une concentration appropriée (par exemple de l'ordre de 10 à 20 µg/ml), pendant une durée suffisante pour permettre la liaison entre les complexes et les cellules T CD4+ spécifiques d'EBV (par exemple, de l'ordre de 1 à 3 heures). Après lavage, la suspension est analysée par cytométrie de flux : on visualise le marquage des cellules par les complexes multimériques qui sont fluorescents.

La cytométrie de flux permet de séparer les cellules marquées par les complexes multimériques HLA II/peptide des cellules non marquées et d'effectuer ainsi un tri cellulaire.

La présente invention a ainsi, en outre, pour objet un procédé de tri de cellules T CD4+ spécifiques d'un antigène de latence d'EBV, **caractérisé en ce qu**'il comprend au moins les étapes suivantes :
- la mise en contact d'un échantillon cellulaire avec des complexes multimériques HLA II/peptide marqués, notamment par un fluorochrome, lesdits complexes étant formés par la liaison de molécules HLA II solubles avec au moins un peptide d'EBV tel que défini ci-dessus, et
- le tri des cellules liées auxdits complexes HLA II/peptide, notamment en cytométrie de flux.

Outre les dispositions qui précèdent, l'invention comprend encore d'autres dispositions, qui ressortiront de la description qui va suivre, qui se réfère à des exemples de mise en oeuvre de l'objet de la présente invention, avec référence au dessins annexés dans lesquels :

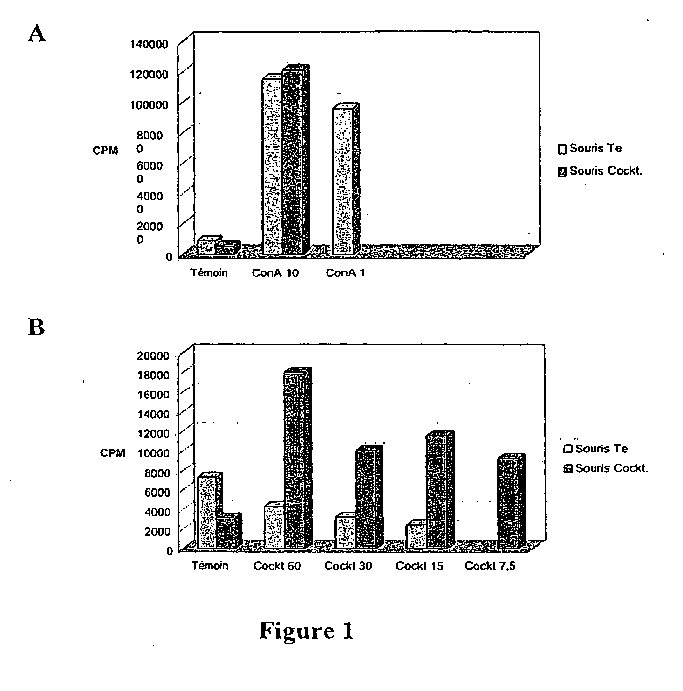

- la figure 1 illustre l'immunogénicité du mélange des six peptides issus des antigènes de latence de type I et II d'EBV se liant avec une affinité < 1000 nM aux 12 molécules HLA II prépondérantes dans la population caucasienne, dans le modèle murin transgénique HLA-DR1. Un groupe de cinq souris HLA- transgéniques Aβ°/DR1 (souris Cockt) a été immnunisé par une première injection sous-cutanée du mélange peptidique (120 µg au total) émulsifié dans du Montanide® Isa 720, suivie de 2 rappels à demi-dose, à 2 semaines d'intervalle. Le groupe témoin (Souris Te), n'a reçu que du Montanide® Isa 720, selon le même protocole. La réponse proliférative des lymphocytes extraits des ganglions, 7 jours après la dernière injection, a été mesurée par incorporation de (³H)thymidine, dans différentes conditions. **A** : en l'absence d'antigène (témoin) ou en présence d'une gamme concanavaline A (ConA) (cultures de 48 heures). **B** : en l'absence d'antigène (témoin) ou en présence d'une gamme de concentrations du mélange peptidique (60, 30, 15 ou 7,5 µg au total), (cultures de 5 jours). Les résultats sont exprimés en nombre de coups par minute (cpm).

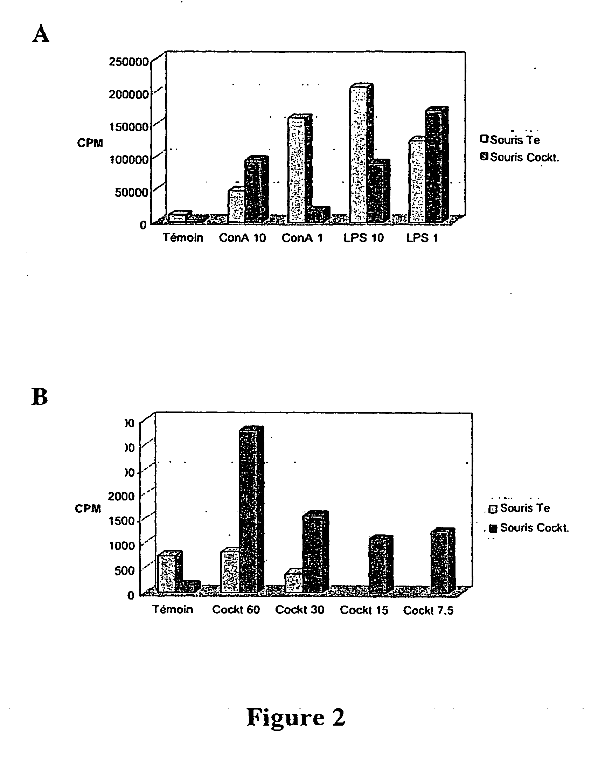

- la figure 2 illustre l'immunogénicité du mélange des six peptides issus des antigènes de latence de type I et II d'EBV se liant avec une affinité < 1000 nM aux 12 molécules HLA II prépondérantes dans la population caucasienne, dans le modèle murin transgénique HLA-DR1. Un groupe de cinq souris HLA- transgéniques Aβ°/DR1 (souris Cockt) a été immnunisé par une première injection sous-cutanée du mélange peptidique (120 µg au total) émulsifié dans du Montanide® lsa 720, suivie de 2 rappels à demie-dose, à 2 semaines d'intervalle. Le groupe témoin (Souris Te), n'a reçu que du Montanide® Isa 720, selon le même protocole. La réponse proliférative des lymphocytes extraits des rates, 7 jours après la dernière injection, a été mesurée par incorporation de (³H)thymidine, dans différentes conditions. **A** : en l'absence d'antigène (témoin) ou en présence d'une gamme concanavaline A (ConA) ou de LPS (cultures de 48 heures). **B** : en l'absence d'antigène (témoin) ou en présence d'une gamme de concentrations du mélange peptidique (60, 30, 15 ou 7,5 µg au total), (cultures de 5 jours). Les résultats sont exprimés en nombre de coups par minute (cpm).

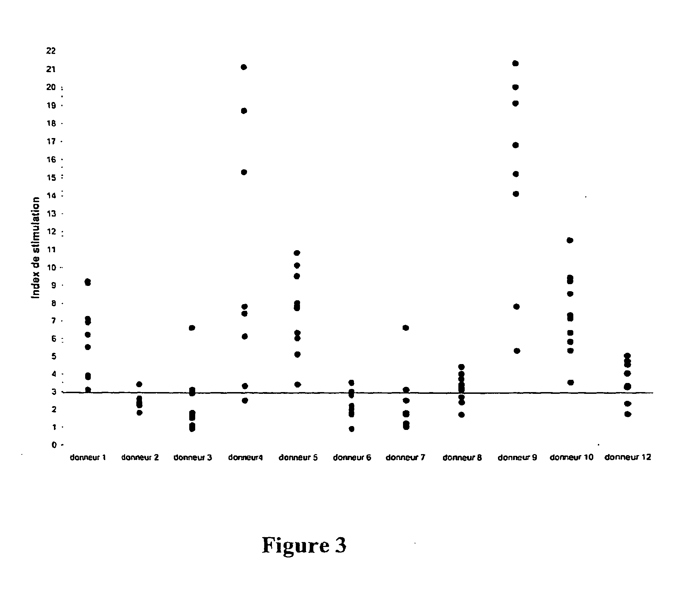

- la figure 3 illustre la réponse proliférative des lymphocytes T issus de donneurs sains d'haplotype HLA II variés, séropositifs pour EBV, après stimulation *in vitro* par le mélange peptidique. Les cellules mononucléées sanguines issues de sujets sains séropositifs pour l'EBV (plaques 96 puits, 5x10⁵ cellules par puits) ont été cultivées 4 à 6 jours en présence du mélange contenant les peptides G17F, N26E, V21V, K26L, P24G et I16L (SEQ ID NO: 21, 4, 20, 5, 6 et 11) à la concentration de 10µg/mL pour chacun d'entre eux. La réponse proliférative des lymphocytes T a été mesurée par incorporation de (³H)thymidine. L'index de stimulation correspondant au rapport du nombre de coups par minute des lymphocytes T en présence du mélange peptidique sur le nombre moyen de coups par minute des lymphocytes T en l'absence de peptide. (bruit de fond), est présenté pour chaque puits pour les différents donneurs. Un puits est considéré comme positif lorsque le nombre de coups par minute est supérieur a 1000 et l'index de stimulation supérieur à 3.

### Exemple 1 : Identification d'épitopes T CD4+ reconnus par les molécules HLA II les plus abondantes dans la population caucasienne.

### 1) Matériels et méthodes

### 1.1) Synthèse peptidique

Le logiciel TEPITOPE (Sturniolo et al., Nature Biotechnology, 1999, 17, 555-561) a été utilisé pour sélectionner des motifs de liaison potentiels aux molécules HLA de classe 11, dans la séquence des antigènes de latence de type II d'EBV (EBNA1, LMP1 et LMP2). Les peptides correspondants ont été synthétisés selon la stratégie Fmoc en synthèse parallèle sur phase solide (Néosystem), purifiés par chromatographie liquide de haute performance (HPLC ; colonne C18, SYNMETRY), puis contrôlés par spectrométrie de masse (ES-MS).

### 1.2) Tests de liaison HLAII/peptide

Les tests de liaisons aux molécules HLA II sont des tests de liaison en compétition avec une révélation immuno-enzymatique dont le principe est décrit dans le Brevet US 6,649,169 pour les molécules HLA-DR et dans la Demande Internationale PCT WO 03/040299 pour les molécules HLA-DP4, ainsi que dans les articles aux noms de Texier et al., J. Immunol., 2000, 164, 3177 et Eur. J. Immunol., 2001, 31, 1837 et Castelli et al., J. Immunol, 2002,169, 6928-6934.

Des exemples de ces tests sont présentés dans les Demandes Internationales PCT WO 02/090382, WO 03/040299 et WO 2004/014936.

### a) Choix des allèles HLA II

12 molécules HLA II (10 molécules HLA-DR et 2 molécules HLA-DP) les plus abondantes dans la population française et dont les fréquences alléliques sont caractéristiques de la population caucasienne, ont été sélectionnées :

### * molécules HLA-DR dont la chaîne β est codée par le gène DR1

II s'agit des molécules HLA-DR1, -DR3, -DR4, -DR7, -DR11, - DR13 et -DR15 dont la chaîne β est codée par les allèles du locus DRB1 dont la fréquence dépasse les 5 % dans la population française : DRB1*0101 (9,3 %), DRB1*0301 (10,9 %), DRB1*0401 (5,6 %), DRB1*0701(14 %), DRB1*1101 (9,2 %), DRB1*1301 (6 %) et DRB1*1501 (8 %) qui représentent à eux seuls 64 % de la population. Ces mêmes allèles sont les allèles HLA-DR les plus abondants dans les autres populations caucasiennes. Leur fréquence varie entre 53 % (en Espagne) et 82 % (au Danemark). Pour les Etats-Unis et le Canada, ils représentent respectivement 58 et 55 % des allèles DR de la population.

### * molécules HLA-DR dont la chaîne β n'est pas codée par le gène DR1

Il s'agit des molécules HLA-DRB3, -DRB4 et -DRB5 dont la chaîne β est codée par les allèles les plus fréquents dans la population française : HLA-DRB3*0101 (9,2 %), HLA-DRB4*0101 (28,4 %), et HLA-DRB5*0101 (7,9 %). Ces molécules couvrent à elles seules 45 % de la fréquence allélique.

### * molécules HLA-DP

II s'agit des molécules HLA-DP4 qui regroupent les molécules codées par les allèles DPB1*0401 et DPB1*0402. Ces molécules DP4 sont les molécules HLA II les plus abondantes en Europe et aux États-Unis. Leur fréquence allélique est en effet de 40 et 11 % respectivement ce qui signifie qu'elles sont l'une ou l'autre trouvées chez environ 76% des individus. Les peptides présents dans une séquence protéique et qui lient l'ensemble de ces molécules incluent donc les épitopes T CD4⁺de la majorité de la population.

### b) Purification des molécules HLA II

Les molécules HLA II sont purifiées par immunoaffinité à partir de différentes lignées homozygotes de lymphocytes B humains transformées par le virus Epstein Barr (EBV), à savoir: HOM2 (DRB1*0101, DPB1*0401), SCHU (DRB1*1501, DRB5*0101, DPB1*0402), STEILIN (DRB1*0301, DRB3*0101), PITOUT (DRB1*0701, DBR4*0101, DPB1*0401), SWEIG (DRB1*1101), BOLETH (DRB1*0401, DRB4*0103), HHKB (DRB1*1301, DRB3*0101, DPB1*0401). Les molécules HLA-DR sont purifiées par chromatographie d'affinité à l'aide de l'anticorps monoclonal monomorphique L243 (Smith et al., P.N.A.S., 1982, 79, 608-612) couplé au gel de protéine A-sépharose CL 4B (PHARMACIA), comme décrit dans Gorga et al., J. Biol. Chem., 1987, 262, 16087-16094. Les molécules HLA-DP4 sont purifiées selon le même protocole, à l'aide de l'anticorps monomorphique B7/21 1 (WATSON et al., Nature, 1983, 304, 358-361). Brièvement, les cellules (5x10⁸ cells/ml) sont lysées sur de la glace, dans du tampon 150 mM NaCl, 10 mM Tris HCl, pH=8,3, contenant 1% Nonidet P40, 10 mg/l aprotinine, 5 mM EDTA et 10 µM PMSF. Après une centrifugation à 100 000 g pendant 1 h, le surnageant est chargé sur les colonnes de sépharose 4B, de protéine A-sépharose 4B, puis sur la colonne d'affinité spécifique. Les molécules HLA II sont éluées avec un tampon 1,1 mM n-dodécyl β-D-maltoside (DM), 500 mM NaCl et 500 mM Na₂CO₃, pH=11,5. Les fractions sont immédiatement neutralisées à pH=7 avec un tampon 2 M Tris HCl, pH=6,8 puis dialysées de façon extensive, contre un tampon 1 mM DM, 150 mM NaCl, 10mM, pH=7.

### c) Synthèse des peptide traceurs

Les peptides traceurs utilisés dans les tests de liaison sont les suivants : HA 306-318 (PKYVKQNTLKLAT, SEQ ID NO : 23), A3 152-166 (EAEQLRAYLDGTGVE, SEQ ID NO: 24), MT 2-16 (AKTIAYDEEARRGLE, SEQ ID NO : 25), YKL (AAYAAAKAAALAA, SEQ LD NO : 26), B1 21-36 (TERVRLVTRHIYNREE, SEQ ID NO: 27), LOL 191-210 (ESWGA VWRJDTPDKL TGPFT, SEQ ID NO : 28), E2/E168 (AGDLLAIETDKATI, SEQ ID NO : 29) et Oxy 271-287 (EKKYFAATQFEPLAARL, SEQ ID NO : 30). Les peptides sont synthétisés selon la stratégie Fmoc en synthèse parallèle sur phase solide, biotinylés au niveau du résidu NH₂ terminal à l'aide d'acide biotinyl-6-aminocaproïque (FLUK-A CHIMIE), selon le protocole décrit dans Texier et al., J. Immunol., 2000, 164, 3177-3184), puis clivés de la résine par de l'acide trifluoroacétique (95 %) et purifiés par chromatographie liquide de haute performance en phase inverse, sur une colonne C₁₈ (Vydac™).

### d) Tests de liaison HLA II/peptides

Les molécules HLA II sont diluées dans du tampon phosphate 10 mM, NaCl 150 mM, dodécyl maltoside (DM) 1 mM, citrate 10 mM, thimérosal 0,003%, en présence d'un peptide biotinylé approprié (peptide traceur), à un pH donné, et de dilution sériées de peptide compétiteur (peptide à tester). A savoir : le peptide HA 306-318 est utilisé à 1 nM et 30 nM, à pH=6, respectivement pour les allèles DRB1*0101 et DRB1*0401 et à 20 nM, à pH=5, pour l'allèle DRB1*1101. YKL est utilisé à 20 nM, à pH=5, pour l'allèle DRB1*0701. L'incubation est réalisée à pH=4,5 pour les allèles DRB1*1501, DRB1*1301 et DRB1*0301, en présence de respectivement, A3 152-166 (10 nM), B1 21-36 (200 nM) et MT 2-16 (200 nM). Le peptide oxybiotinylé est utilisé à pH=5 (10 nM) pour les allèles DPB1*0401 et DPB1*0402. Le mélange réactionnel (100 µl par puits) est incubé dans des plaques à 96 puits en polypropylène (NUNC), à 37°C pendant 24 h, à l'exception des allèles DRB1*1301 et DRB1*0301 qui sont incubés pendant 72 h. A la fin de l'incubation, les échantillons sont neutralisés avec 50 µL de tampon Tris HCl 450 mM, pH 7,5, thimérosal 0,003 %, BSA 0,3 %, DM 1 mM. Les échantillons sont ensuite transférés sur des plaques ELISA Maxisorp™ (96 puits, NUNC) prélablement recouvertes d'anticorps L243 ou B7/21 L243 (10 µg/ml) et saturées avec du tampon Tris HCl 100 mM, pH=7.5, BSA 0,3 %, thimerosal 0,003%. L'incubation des échantillons sur ces plaques est réalisée pendant deux heures à température ambiante. Des lavages avec du tampon Tris HCl 0,1M, pH 7,5, Tween-20 0,05% sont ensuite effectuées entre chaque étape. Le peptide biotinylé lié aux molécules HLA II est détecté par l'addition de 100 µL/puits du conjugué streptavidine-phosphatase alcaline (45 minutes, AMERSHAM) dilué au 1/2000 dans le tampon Tris 10 mM, pH 7, NaCl 0,15 M, Tween 20 0,05%, BSA 0,2%, thimérosal 0,003%, puis de 200 µL/puits du substrat 4-méthyl-umbelliféryl-phosphate (MUP, SIGMA) à la concentration de 100 µM, en tampon NaHCO₃ 0,05M, pH 9,8, MgCl₂ 1mM. L'émission de fluorescence par le produit de la réaction enzymatique est mesurée à 450 nm après excitation à 365 nm, à l'aide d'un fluorimètre pour plaques à 96 puits (DYNEX). La liaison maximale est déterminée en incubant le peptide traceur biotinylé avec la molécule HLA II en l'absence de peptide compétiteur. La spécificité de liaison est contrôlée par l'addition d'un excès de peptide non biotinylé. Le bruit de fond obtenu ne diffère pas significativement de celui obtenu en incubant le peptide biotinylé sans les molécules IILA II. Les résultats sont exprimés sous la forme de la concentration de peptide compétiteur qui inhibe 50% de la liaison maximale du peptide traceur biotinylé (IC₅₀). Les moyennes et les écarts-types sont calculés à partir d'au moins trois expériences indépendantes. La validité de chaque expérience est déterminée à l'aide des peptides traceurs ; leurs valeurs d'IC₅₀ varient d'un facteur inférieur à trois. Un peptide dont la valeur d'IC₅₀ est inférieure à 1000 mM est considéré comme possédant une bonne affinité pour la molécule HLA II correspondante.

### 2) Résultats

L'analyse des séquences des antigènes EBNA1, LMP1 et LMP2 a permis d'identifier 24 séquences peptidiques de 15 à 31 acides aminés, susceptibles de contenir des motifs pour plusieurs molécules HLA-DR. Parmi ces 24 peptides, 22 ont pu être synthétisés en chimie f-moc avec une pureté de l'ordre de 90% en HPLC. Neuf peptides sont dérivés de EBNA1, 7 de LMP1 et 6 de LMP2. Les séquences des peptides dérivés des séquence présentant les numéros d'accès NP_039875, CAA26023 et AAA45886, respectivement pour EBNA1, LMP1 et LMP2), sont présentées dans la liste de séquences jointe en annexe et le Tableau II.

**Tableau II: Séquences des peptides**

| Peptide | Position | Séquence | Numéro d'identification |
|---|---|---|---|
| EBNA1 | | | |
| R19T | 67-85 | RDGVRRPQKRPSCIGCKGT | SEQ ID NO : 1 |
| R26D | 403-428 | RPFFHPVGEADYFEYHOEGGPDGEPD | SEQ ID NO : 2 |
| D22P | 455-476 | OGGRRKKGGWFGKHRGOGGSNP | SEQ ID NO : 3 |
| N26E | 475-500 | NPKFENIAEGLRALLARSHVERTTDE | SEQ ID NO : 4 |
| K26L | 514-539 | KTSLYNLRRGTALAIPQCRLTPLSRL | SEQ ID NO : 5 |
| P24G | 529-552 | PQCRLTPLSRLPFGMAPGPGPOPG | SEQ ID NO : 6 |
| M23T | 563-585 | MVFLQTHIFAEVLKDAIKDLVMT | SEQ ID NO : 7 |
| F24R | 571-594 | FAEVLKDAIKDLVMTKPAPTCNIR | SEQ ID NO : 8 |
| L19G | 606-624 | LPPWFPPMVEGAAAEGDDG | SEQ ID NO :9 |

| LMP1 | | | |
|---|---|---|---|
| E15P | 2 à 16 | EHDLERGPPGPRRPP | SEQ ID NO: 10 |
| 116L | 68-83 | IIFIFRRDLLCPLGAL | SEQ ID NO: 11 |
| A16L | 96-111 | ALWNLHGQALFLGIVL | SEQ ID NO : 12 |
| A17H | 179-195 | AILIWMYYHGPRHSDEH | SEO ID NO : 13 |
| N15D | 303-317 | HDPLPHSPSDSAGND | SEQ ID NO : 14 |
| S27L | 211 à 237 | SGHESDSNSNEGRHHLLVSGAGDGPPL | SEG1 ID NO : 15 |
| D31D | 356 à 386 | DPHLPTLLLGTSGSGGDDDDPHGPVQLSYYD | SEQ ID NO : 16 |

| LMP2 | | | |
|---|---|---|---|
| M20D | 1 à 20 | MGSLEMVPMGAGPPSPGGDP | SEQ ID NO : 17 |
| E25Y | 61 à 85 | EDPYWGNGDRHSDYOPLGTQDOSLY | SEQ ID NO : 18 |
| A15V | 148-163 | ASSYAAAQRKLLTPV | SEQ ID NO : 19 |
| V21V | 224-244 | VLVMLVLLILAYRRRWRRLTV | SEQ ID NO : 20 |
| G17F | 372-388 | GGSILQTNFKSLSSTEF | SEQ ID NO: 21 |
| L18L | 410-427 | LTEWGSGNRTYGPVFMCL | SEQ ID NO : 22 |

L'activité de liaison des peptides vis-à-vis des 12 molécules HLA II prépondérantes dans la poplulation caucasienne (HLA-DR1,-DR3,-DR4,-DR7,-DR11, -DR13,-DR15,-DRB3,-DRB4, -DRB5, DP401 et -DP402 ; Tableau III) montre que 6 peptides se lient avec une bonne affinité à au moins 7 molécules HLA II différentes : **G17F** (LMP2), **N26E** (EBNA1), **V21V** (LMP2), **K26L**(EBNA1), **P24G** (EBNA1), **I16L** (LMP1).

**Tableau III: Activité de liaison des peptides vis-à-vis des molécules HLA II prépondérantes dans la population caucasienne.**

| IC₅₀ *(nM) | | | | | | | | | | | | | | |
|---|---|---|---|---|---|---|---|---|---|---|---|---|---|---|
| Peptide | DR1 | DR3 | DR4 | DR7 | DR11 | DR13 | DR15 | DRB3 | DRB4 | DRB5 | DP401 | DP402 | Nombre d'allèles liés | Classement |
| L18L | Insoluble | Insoluble | >100000 | 1061 | >100000 | Insoluble | Insoluble | Insoluble | 2100 | Insoluble | Insoluble | Insoluble | 0 | 17 |
| **G17F** | **15** | **851** | **335** | **40** | **29** | Insoluble | **28** | > 100000 | Insoluble | **170** | **16** | **25** | 9 | **2** |
| **N26E** | **21** | 8485 | **235** | **184** | **447** | 1649 | **229** | >100000 | **825** | **17** | 4094 | 4402 | 7 | **4** |
| A17H | 7746 | 7348 | >100000 | >100000 | >100000 | Insoluble | **3** | >100000 | **150** | **42** | >100000 | >100000 | 3 | **8** |
| A16L | 79 | Insoluble | >100000 | **122** | 10954 | Insoluble | **48** | >100000 | >100000 | 2683 | 1182 | **725** | 4 | 7 |
| M20P | **721** | >100000 | 7483 | >100000 | 4243 | >100000 | 4690 | >100000 | 6735 | 1039 | 11745 | 10595 | 1 | 12 |
| L19G | 6221 | >100000 | >100000 | 18166 | 1095 | >100000 | 69354 | >100000 | >100000 | 37417 | >100000 | 30984 | 0 | 17 |
| S27L | >100000 | >100000 | >100000 | 18000 | 19339 | >100000 | 45000 | >100000 | >100000 | 13477 | >100000 | >100000 | 0 | 17 |
| D31D | 17321 | >100000 | >100000 | 27495 | 25000 | >100000 | **251** | >100000 | >100000 | >100000 | >100000 | 28284 | 1 | 12 |
| A15V | 1196 | >100000 | 35496 | 1732 | **4** | >100000 | 26458 | >100000 | >100000 | **463** | >100000 | >100000 | 2 | 9 |
| E25Y | 949 | >100000 | 5099 | 9798 | 2258 | >100000 | 1285 | >100000 | >100000 | >100000 | >100000 | >100000 | 1 | 12 |
| V21V | **240** | **77** | 390 | **71** | 130 | **161** | **693** | **417** | **167** | **169** | **150** | **155** | **12** | 1 |
| RT19 | **698** | >100000 | 9899 | 38536 | 11292 | >100000 | 7266 | >100000 | >100000 | **771** | >100000 | >100000 | 2 | 9 |
| **K26L** | 1,1 | 60000 | **346** | **1,7** | **1** | 2049 | **24** | >100000 | **200** | **128** | **194** | **339** | 9 | 2 |
| R26D | 3742 | >100000 | 20494 | 1129 | >100000 | >100000 | 22361 | 3969 | 79373 | 5477 | >100000 | >100000 | 0 | 17 |
| **P24G** | 47 | 32787 | 2627 | 116 | **2** | **775** | 2621 | 31464 | 5532 | 419 | **120** | **447** | 7 | 4 |
| F24R | **65** | 1936 | 1500 | **56** | 1554 | >100000 | 3464 | 16125 | 63246 | 5399 | 17544 | 2739 | 2 | 9 |
| D22P | 62450 | >100000 | >100000 | 86603 | **229** | >100000 | >100000 | >100000 | >100000 | >100000 | >100000 | >100000 | 1 | 12 |
| **I16L** | **190** | **110** | 7348 | **175** | **27** | >100000 | **9** | **224** | **529** | >100000 | >100000 | 1111 | 7 | 4 |
| E15P | >100000 | >100000 | >100000 | >100000 | >100000 | >100000 | >100000 | >100000 | >100000 | 5254 | >100000 | >100000 | 0 | 17 |
| H15D | >100000 | >100000 | >100000 | >100000 | >100000 | >100000 | >100000 | >100000 | >100000 | >100000 | >100000 | >100000 | 0 | 17 |

| | | | | | | | | | | | | | | |
|---|---|---|---|---|---|---|---|---|---|---|---|---|---|---|
| * un peptide possédant une vaieui-d'IC₅₀ intérieure à 1000 nM est considéré comme possédant une bonne affinité pour la molécule HLA II correspondante | | | | | | | | | | | | | | |

Compte tenu de la fréquence allélique de chacun des 12 allèles HLA II testés, un mélange comportant ces 6 peptides serait capable d'être reconnu par plus de 90% de la population caucasienne.

### Exemple 2 : Immumogénicité des peptides in vivo chez des souris transgéniques pour une molécule HLA II

La capacité des peptides ayant une bonne affinité pour les molécules HLA II, tels que définis à l'exemple 1 à induire une réponse immunitaire, a été évaluée par un test de prolifération *in vitro,* chez des souris transgénique pour les molécules HLA-DR1 (souris transgéniques Aβ°/DR1 ; Pancré et al., Clinical and Experimental Immunology, 2002 ; 129: 429-437).

### 1) Matériels et méthodes

Un premier groupe de souris transgéniques âgées de 6 semaines, a été immunisé par une première injection sous-cutanée du mélange peptidique déterminé à l'exemple 1 (20 µg de chaque peptide, soit 120 µg au total) émulsifié dans du Montanide® Isa 720 (SEPPIC), suivie de deux rappels à 2 semaines d'intervalle, avec une demi-dose du mélange peptidique (10 µg de chaque peptide, 60 µg au total) émulsifié dans du Montanide® Isa 720. Le groupe témoin a reçu uniquement des injections de Montanide® Isa 720, selon le même protocole. Sept jours après la dernière injection, les animaux ont été sacrifiés et la rate ainsi que des ganglions lymphatiques ont été prélevés. Les cellules de rate ou de ganglion lymphatique (5.10⁵/puits d'une plaque de microtitration de 96 puits (Falcon®), en triplicat), ont été mises en culture, soit en l'absence d'antigène (témoin négatif), soit en présence d'une gamme de LPS (Sigma®, 10 mg et 1 mg) ou de concanavaline A (Sigma®, 10 mg et 1 mg, (témoins positifs, cultures de 48h), soit en présence d'une gamme de concentrations (60 µg, 30 µg, 15 µg, 7,5 µg au total) du mélange peptidique (cultures de 5 jours). Après la culture, de la thymidine tritiée ((³H)thymidine) (1 mCi/puits, 49,0 Ci/mmol, AMERSHAM) a été ajoutée et les cellules ont été récupérées 6 heures plus tard par aspiration sur filtre en fibre de verre (WALLAC). La thymidine incorporée a été mesurée à l'aide d'un compteur de scintillation β (1450 Trilux, WALLAC). Les résultats sont exprimés en index de prolifération (nombre de coups par minute (CPM) en présence d'antigène/nombre de CPM en l'absence d'antigène).

### 2) Résultats

Les résultats présentés aux figures 1 et 2 montrent que les peptides sélectionnés par le test de liaison aux molécules HLA II sont capables d'induire une forte réponse CD4+ spécifique d'EBV chez les individus immunisés. Après restimulation *in vitro* avec le mélange peptidique, il existe une réponse proliférative des lymphocytes de la rate et des ganglions extraits des animaux vaccinés. En revanche, cette prolifération n'est pas observée chez les animaux contrôles non immunisés (figures 1 et 2). Ces résultats indiquent donc que les peptides sélectionnés peuvent être utilisés dans des compositions immunogènes pour la prévention et le traitement des pathologies associées aux infections par EBV.

### Exemple 3: Immunogénicité des peptides in vitro chez des sujets sains séropositifs pour EBV

### 1) Matériels et méthodes

### a) Donneurs et typage HLA

Du sang a été prélevé chez 12 donneurs volontaires sains séropositifs pour l'EBV d'haplotype HLA 11 varié.

**Tableau IV: Haplotypes HLA-DRB1 des donneurs volontaires sains séropositifs pour 1' EBV**

| **Donneur** | **Haplotype HLA-DRB1** |
|---|---|
| 1 | DRB1*1101/ DRB1*1301 |
| 2 | DRB1*0401/ DRB1*1201 |
| 3 | DRB1*1501/ DRB1*0301 |
| 4 | DRB1*0701/ DRB1*1101 |
| 5 | DRB1*0101/ DRB1*1101 |
| 6 | DRB1*1501/ DRB1*0401 |
| 7 | DRB1*0301/ DRB1*1301 |
| 8 | DRB1*1101/ DRB1*1201 |
| 9 | DRB1*1401/ DRB1*0701 |
| 10 | DRB1*1101/ DRB1*1201 |
| 11 | DRB1*0401/ DRB1*1101 |
| 12 | DRB1*0401/ DRB1*0801 |

### b) Réponse proliférative des lymphocytes T au mélange peptidique ou aux peptides testés individuellement

Les cellules mononucléées du sang (PBMCs) des donneurs ont été séparées par la méthode classique du gradient de Ficoll. Les PBMCs (5.10⁵ cellules/puits d'une plaque à 96 puits) ont ensuite été cultivées 4 à 6 jours dans un milieu sans sérum (AIM V, GIBCO^{™}), en présence ou en l'absence du mélange peptidique (60 µg/mL au total, soit 10µg/mL de chaque peptide). La réponse proliférative a été mesurée par incorporation de (³H)thymidine (1 mCi/puits, 49,0 Ci/mmol, AMERSHAM). Les résultats sont exprimés en index de stimulation (coups par minute des lymphocytes T stimulés/moyenne des coups par minute des lymphocytes T non stimulés). Les puits correspondant aux lymphocytes T stimulés ont été analysés de manière individuelle et considérés connme positifs lorsque le nombre de coups par minute était supérieur à 1000 et l'index de stimulation supérieur à 3 (nombre de coups par minute en présence du mélange de peptide 3 fois supérieur à celui observé en l'absence de peptides (bruit de fond)). Les peptides ont également été testés de manière individuelle chez 6 donneurs (donneurs 1, 3, 5, 6, 9 et 10). Pour ce faire, une gamme de concentrations (2,5, 5, 10 et 20 µg) a été testée en triplicat pour chaque peptide. La réponse a été considérée comme positive lorsque la moyenne des index de stimulation des triplicats était supérieure à 2 avec un puits possédant un index de stimulation supérieur a 3.

### c) Détection de cytokines

Les PBMCs des donneurs (1.10⁶ cellules/puits, plaques 24 puits) ont été cultivés dans du mileu AIM V (GIBCO^{™}) ou RPMI 1640 (GIBCO^{™}), supplémenté avec 10% de sérum humain de groupe AB, en présence ou en l'absence du mélange peptidique (60 µg/mL), pendant 2 ou 5 jours. L'IL-2, 1' IL-4, 1'IL-10 et 1'IFN-γ présents dans les surnageants de culture ont été dosés en ELISA sandwich, selon un protocole standard, à l'aide d'anticorps dirigés contre chacune de ces cyrtoines (BD PharMingen). L'absorbance à 492 nm a ete mesurée à l'aide d'un spectrophotomètre (Multiskan RC, LABSYSTEMS). Les résultats présentés correspondent à la moyenne obtenus sur deux puits.

### 2) Résultats

### a) réponse T CD4+ spécifique d'EBV induite par le mélange peptidique

Afin de déterminer si le mélange peptidique était reconnu par les lymphocytes T CD4+ humains, des cellules mononucléées sanguines issues de donneurs volontaires sains séropositifs pour EBV, d'haplotype HLA varié (Tableau III) ont été stimulées *in vitro* par le mélange peptidique. Dans un premier temps, la réponse proliférative des lymphocytes T qui permet d'établir l'existence d'une réponse lymphocytaire T de type mémoire, a été évaluée par mesure de l'incorporation de (³H)thyrnidine après 4 à 6 jours de culture en présence du mélange peptidique. La réponse proliférative a pu être évaluée chez 11 donneurs (figure 3). Tous les donneurs montrent une réponse proliférative, avec des niveaux de prolifération variables d'un donneur à l'autre. Ces résultats indiquent la présence de lymphocytes T mémoires capables de reconnaître le mélange peptidique, chez l'ensemble des donneurs testés. Les résultats indiquent également que le mélange contient des peptides naturellement présentés au cours de l'infection par EBV.

La réponse à chacun des peptides testé séparément, a été évaluée (Tableau V). Les résultats confirment l'intérêt de chacun des 6 peptides dans le mélange sélectionné.

**Tableau V : Analyse de la réponse T CD4+ spécifique d'EBV induite par chacun des peptides testé séparément.**

| **Donneurs** | **Peptides** | | | | | | |
|---|---|---|---|---|---|---|---|
| | **EBNA1** | | | **LMP1** | **LMP2** | | |
| | N26E | K26L | P24G | I16L | G17F | V21V | |
| **1** | + | + | - | - | - | - | **2/6** |
| **3** | + | + | - | + | - | + | **4/6** |
| **5** | + | + | + | + | - | - | **4/6** |
| **6** | + | + | + | + | + | + | **6/6** |
| **9** | + | + | + | + | + | + | **6/6** |
| **10** | - | + | + | + | + | + | **5/6** |
| | **5/6** | **6/6** | **4/6** | **5/6** | **3/6** | **4/6** | |

La sécrétion de cytokines dans le milieu de culture après stimulation des PBMCs avec le mélange peptidique, a également été testée en ELISA (Tableau VI).

**Tableau VI : Analyse de la sécrétion de cytokines après stimulation avec le mélange peptidique.**

| | **Cytokines (pg/mL)** | | | | | | | |
|---|---|---|---|---|---|---|---|---|
| | **IL-2** | | **IL-4** | | **IL-10** | | **IFN-γ** | |
| | AIM* | SAB** | AIM | SAB | AIM | SAB | AIM | SAB |
| Donneur 1 | - | - | - | - | - | 80 | - | - |
| Donneur 2 | - | - | - | - | - | 290 | - | 211 |
| Donneur 3 | - | - | - | - | - | 645 | - | - |
| Donneur 4 | - | - | - | - | - | - | - | - |
| Donneur 5 | - | - | - | - | - | - | - | 220 |
| Donneur 6 | - | - | - | - | - | - | 409 | 583 |
| Donneur 7 | - | - | - | - | - | - | - | - |
| Donneur 8 | - | - | - | - | - | 183 | - | - |
| Donneur 9 | - | - | - | - | - | - | - | 268* |
| Donneur 10 | - | - | - | - | - | - | 84 | 541 |
| Donneur 11 | - | - | - | - | - | - | 329 | 52 |
| Donneur 12 | - | - | - | - | - | - | 233 | - |

| | | | | | | | | |
|---|---|---|---|---|---|---|---|---|
| * PBMC cultivés en milieu AIM ** PBMC cultivés en milieu RPMI+sérum humain de groupe AB | | | | | | | | |

Une sécrétion significative d'IFN-γ a pu être mise en évidence chez 7 des 12 donneurs. Une sécrétion d'IL-10 a également été retrouvée chez 4 des 12 donneurs. Aucune production significative d'IL-4 ou d'II-2 n'a été retrouvée par ELISA. Par comparaison, les contrôles en l'absence de mélange peptidique étaient négatifs, à l'exception d'un cas (*: IFN-γ retrouvé à 12b pg/mL).

## Revendications

1. Peptide immunogène du virus Epstein-Barr (EBV), **caractérisé en ce qu'**il comprend au moins un épitope T CD4+ de l'un des antigènes de latence EBNA1, LMP1 ou LMP2, apte à être présenté par au moins 7 molécules HLA II différentes choisies parmi les molécules HLA-DR1, HLA-DR3, HLA-DR4, HLA-DR7, HLA-DR11, HLA-DR13, HLA-DR15, HLA-DRB3, HLA-DRB4, HLA-DRB5 et HLA-DP4, et **en ce qu'**il est sélectionné dans le groupe constitué par :
a) les peptides de respectivement 26, 26 et 24 acides aminés dont la séquence s'étend des positions 475 à 500, 514 à 539, et 529 à 552 d'EBNA1,
b) le peptide de 16 acides aminés dont la séquence s'étend des positions 68 à 83 de LMP1, et
c) les peptides de respectivement 21 et 17 acides aminés dont la séquence s'étend des positions 224 à 244 et 372 à 388 de LMP2.

2. Peptide selon la revendication 1, **caractérisé en ce que** lesdites molécules HLA II comprennent une chaîne β codée respectivement par les allèles DRB1*0101 (molécule HLA-DR1), DRB1*0301 (molécule HLA-DR3) DRB1*0401 (molécule HLA-DR4) DRB1*0701 (molécule HLA-DR7), DRB1*1101 (molécule HLA-DR11), DRB1*1301 (molécule HLA-DR13), DRB1*1501 (molécule HLA-DR15), DRB3*0101 (molécule HLA-DRB3), DRB5*0101 (molécule HLA-DRB5), DP*0401 ou DP*0402 (molécule HLA-DP4).

3. Peptide selon la revendication 1 ou 2, **caractérisé en ce qu'**il est sélectionné dans le groupe constitué par les séquences SEQ ID NO : 4, 5, 6, 11, 20 et 21.

4. Dérivé polyépitopique du peptide selon l'une quelconque des revendications 1 à 3, **caractérisé en ce qu'**il comprend la séquence dudit peptide et la séquence d'au moins un autre épitope identique ou différent.

5. Dérivé polyépitopique selon la revendication 4, **caractérisé en ce qu'**il comprend un épitope T CD4+ dont la séquence est celle du peptide selon l'une quelconque des revendications 1 à 3 et au moins un autre épitope sélectionné dans le groupe constitué par :
- un épitope T CD8+ d'un antigène d'EBV,
- un épitope B d'un antigène d'EBV, et
- un épitope T CD4+ universel naturel ou synthétique tel que le peptide TT 830-846, PADRE, le peptide 45-69 de la protéine Nef du VIH-1 et les peptides CS.T3, CSP, SSP2, LSA-1 et EXP-1.

6. Protéine de fusion, **caractérisée en ce qu'**elle est constituée par une protéine ou un fragment de protéine, fusionné à un peptide selon l'une quelconque des revendications 1 à 3 ou un dérivé polyépitopique selon la revendication 4 ou la revendication 5.

7. Protéine de fusion selon la revendication 6, **caractérisée en ce que** ladite protéine ou ledit fragment est sélectionné dans le groupe constitué par une chaîne alpha ou béta d'une molécule HLA II choisie parmi les molécules HLA-DR1, HLA-DR3, HLA-DR4, HLA-DR7, NLA-DR11, HLA-DR13, HLA-DR15, HLA-DRB3, HLA-DRB4, HLA-DRB5 et HLA-DP4 ou le domaine extracellulaire de ladite chaîne correspondant à une molécule HLA II soluble.

8. Lipopeptide, **caractérisé en ce qu'**il est obtenu par addition d'un lipide sur une fonction a-aminée ou une fonction réactive de la chaîne latérale d'un peptide selon l'une quelconque des revendications 1 à 3 ou d'un dérivé polyépitopique selon la revendication 4 ou la revendication 5.

9. Polynucléotide, **caractérisé en ce qu'**il code pour un peptide, un dérivé polyépitopique ou une protéine de fusion selon l'une quelconque des revendications 1 à 7.

10. Vecteur d'expression, **caractérisé en ce qu'**il comprend un polynucléotide selon la revendication 9 sous le contrôle de séquences régulatrices appropriées, de la transcription et éventuellement de la traduction.

11. Cellule hôte, **caractérisée en ce qu'**elle est modifiée par un polynucléotide selon la revendication 9 ou un vecteur d'expression selon la revendication 10.

12. Composition immunogène ou vaccinale, **caractérisée en ce qu'**elle comprend au moins un peptide, un dérivé polyépitopique, un lipopeptide ou un vecteur d'expression selon l'une quelconque des revendications 1 à 10 et un véhicule pharmaceutiquement acceptable, et/ou une substance porteuse, et/ou un adjuvant.

13. Composition immunogène selon la revendication 12, **caractérisée en ce qu'**elle comprend au moins deux des peptides d'EBV selon l'une quelconque des revendications 1 à 3, de préférence trois à cinq peptides, de manière préférée les six peptides selon la revendication 3, sous forme d'un mélange de peptides ou de lipopeptides tels que définis à la revendication 8, d'un dérivé polyépitopique comprenant lesdits peptides selon la revendication 4 ou la revendication 5 ou d'un vecteur d'expression codant lesdits peptides ou ledit fragment selon la revendication 10.

14. Composition immunogène selon la revendication 13, **caractérisée en ce qu'**elle comprend également, au moins un peptide incluant un épitope sélectionné dans le groupe constitué par un épitope T CD8+ d'EBV, un épitope B d'EBV et un épitope T CD4+ universel, tels que définis à la revendication 5.

15. Peptide, dérivé polyépitopique, lipopeptide ou vecteur selon l'une quelconque des revendications 1 à 5, 8 et 10 comme vaccin pour la prévention et/ou le traitement d'une infection par EBV ou d'une pathologie tumorale associée.

16. Utilisation d'un peptide, d'un dérivé polyépitopique, d'un lipopeptide ou d'un vecteur selon l'une quelconque des revendications 1 à 5, 8 et 10, pour la préparation d'un vaccin destiné à la prévention et/ou au traitement d'une infection par EBV ou d'une pathologie tumorale associée.

17. Réactif de diagnostic d'une infection par EBV ou d'urne pathologie tumorale associée, **caractérisé en ce qu'**il comprend au moins un peptide selon l'une quelconque des revendications 1 à 3, éventuellement marqué où complexé, notamment complexé à des molécules HLA II marquées, sous la forme de complexes multimériques.

18. Utilisation d'un peptide selon l'une quelconque des revendications 1 à 3, pour la préparation d'un réactif de diagnostic d'une infection par EBV ou d'une pathologie tumorale associée.

19. Méthode de diagnostic d'une infection par EBV ou d'une pathologie tumorale associée, **caractérisée en ce qu'**elle comprend :
- la mise en contact d'un échantillon biologique dudit individu avec un réactif de diagnostic selon la revendication 17, et
- la détection de cellules T CD4+ spécifiques d'un des antigènes de latence EBNA1, LMP1 ou LMP2 d'EBV.

20. Procédé de tri de cellules T CD4+ spécifiques d'un des antigènes de latence EBNA1, LMP1 ou LMP2 d'EBV, **caractérisé en ce qu'**il comprend au moins les étapes suivantes :
- la mise en contact d'un échantillon cellulaire avec des complexes multimériques marqués, formés par la liaison de molécules HLA II solubles avec au moins un peptide selon l'une quelconque des revendications 1 à 3, et
- le tri des cellules liées auxdits complexes HLA II/peptide.

## Claims

1. Immunogenic peptide of the Epstein-Barr virus (EBV), **characterised in that** it comprises at least one T CD4+ epitope of one of the latency antigens EBNA1, LMP1 or LMP2, capable of being presented by at least 7 different HLA II molecules selected from the molecules HLA-DR1, HLA-DR3, HLA-DR4, HLA-DR7, HLA-DR11, HLA-DR13, HLA-DR15, HLA-DRB3, HLA-DRB4, HLA-DRB5 and HLA-DP4, and **in that** it is selected from the group consisting of:
a) the peptides of 26, 26 and 24 amino acids whose sequence extends from positions 475 to 500, 514 to 539 and 529 to 552 of EBNA1,
b) the peptide of 16 amino acids whose sequence extends from positions 68 to 83 of LMP1, and
c) the peptides of 21 and 17 amino acids whose sequence extends from positions 224 to 244 and 372 to 388 of LMP2.

2. Peptide according to claim 1, **characterised in that** said HLA II molecules comprise a β chain coded for by the alleles DRB1*0101 (HLA-DR1 molecule), DRB1*0301 (HLA-DR3 molecule), DRB1*0401 (HLA-DR4 molecule), DRB1*0701 (HLA-DR7 molecule), DRB1*1101 (HLA-DR11 molecule), DRB1*1301 (HLA-DR13 molecule), DRB1*1501 (HLA-DR15 molecule), DRB3*0101 (HLA-DRB3 molecule), DRB5*0101 (HLA-DRB5 molecule), DP*0401 or DP*0402 (HLA-DP4 molecule).

3. Peptide according to claim 1 or 2, **characterised in that** it is selected from the group consisting of the sequences SEQ ID NO: 4, 5, 6, 11, 20 and 21.

4. Polyepitope derivative of the peptide according to any one of claims 1 to 3, **characterised in that** it comprises the sequence of said peptide and the sequence of at least one other identical or different epitope.

5. Polyepitope derivative according to claim 4, **characterised in that** it comprises a T CD4+ epitope whose sequence is that of the peptide according to any one of claims 1 to 3, and at least one other epitope selected from the group consisting of:
- a T CD8+ epitope of an EBV antigen,
- a B epitope of an EBV antigen, and
- a natural or synthetic universal T CD4+ epitope such as the peptide TT 830-846, PADRE, the peptide 45-69 of the Nef protein of HIV-1 and the peptides CS.T3, CSP, SSP2, LSA-1 and EXP-1.

6. Fusion protein, **characterised in that** it consists of a protein or a protein fragment, fused to a peptide according to any one of claims 1 to 3 or a polyepitope derivative according to claim 4 or claim 5.

7. Fusion protein according to claim 6, **characterised in that** said protein or said fragment is selected from the group consisting of an alpha or beta chain of an HLA II molecule selected from the molecules HLA-DR1, HLA-DR3, HLA-DR4, HLA-DR7, HLA-DR11, HLA-DR13, HLA-DR15, HLA-DRB3, HLA-DRB4, HLA-DRB5 and HLA-DP4 or the extracellular domain of said chain corresponding to a soluble HLA II molecule.

8. Lipopeptide, **characterised in that** it is obtained by addition of a lipid to an α-aminated functional group or a reactive functional group of the side chain of a peptide according to any one of claims 1 to 3 or of a polyepitope derivative according to claim 4 or claim 5.

9. Polynucleotide, **characterised in that** it codes for a peptide, a polyepitope derivative or a fusion protein according to any one of claims 1 to 7.

10. Expression vector, **characterised in that** it comprises a polynucleotide according to claim 9 under the control of suitable regulatory sequences of transcription and, optionally, of translation.

11. Host cell, **characterised in that** it is modified by a polynucleotide according to claim 9 or an expression vector according to claim 10.

12. Immunogenic or vaccinal composition, **characterised in that** it comprises at least one peptide, polyepitope derivative, lipopeptide or expression vector according to any one of claims 1 to 10 and a pharmaceutically acceptable carrier and/or a carrier substance and/or an adjuvant.

13. Immunogenic composition according to claim 12, **characterised in that** it comprises at least two of the EBV peptides according to any one of claims 1 to 3, preferably from three to five peptides, more preferably the six peptides according to claim 3, in the form of a mixture of peptides or lipopeptides as defined in claim 8, of a polyepitope derivative comprising said peptides according to claim 4 or claim 5 or of an expression vector coding for said peptides or said fragment according to claim 10.

14. Immunogenic composition according to claim 13, **characterised in that** it also comprises at least one peptide including an epitope selected from the group consisting of a T CD8+ epitope of EBV, a B epitope of EBV and a universal T CD4+ epitope, as defined in claim 5.

15. Peptide, polyepitope derivative, lipopeptide or vector according to any one of claims 1 to 5, 8 and 10 as a vaccine for the prevention and/or treatment of an EBV infection or an associated tumour pathology.

16. Use of a peptide, polyepitope derivative, lipopeptide or vector according to any one of claims 1 to 5, 8 and 10 for the preparation of a vaccine for the prevention and/or treatment of an EBV infection or an associated tumour pathology.

17. Diagnostic reagent for an EBV infection or an associated tumour pathology, **characterised in that** it comprises at least one peptide according to any one of claims 1 to 3, optionally labelled or complexed, especially complexed with labelled HLA II molecules, in the form of multimeric complexes.

18. Use of a peptide according to any one of claims 1 to 3 for the preparation of a diagnostic reagent for an EBV infection or an associated tumour pathology.

19. Method for diagnosing an EBV infection or an associated tumour pathology, **characterised in that** it comprises:
- bringing a biological sample of said individual into contact with a diagnostic reagent according to claim 17, and
- detecting T CD4+ cells specific for one of the latency antigens EBNA1, LMP1 or LMP2 of EBV.

20. Method of screening T CD4+ cells specific for one of the latency antigens EBNA1, LMP1 or LMP2 of EBV, **characterised in that** it comprises at least the following steps:
- bringing a cell sample into contact with labelled multimeric complexes formed by binding soluble HLA II molecules to at least one peptide according to any one of claims 1 to 3, and
- selecting the cells bonded to said HLA II/peptide complexes.

## Patentansprüche

1. Immunogenes Epstein-Barr-Virus (EBV)-Peptid, **dadurch gekennzeichnet, dass** es wenigstens ein CD4+-T-Epitop von einem der Latenzantigene EBNA1, LMP1 oder LMP2 umfasst, die von wenigstens 7 verschiedenen HLA 11-Molekülen präsentiert werden können, die ausgewählt sind aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5 und HLA-DP4, und **dadurch**, dass es ausgewählt ist aus der Gruppe bestehend aus:
a) den Peptiden mit 26, 26 und 24 Aminosäuren, deren Sequenz sich an Position 475 bis 500, 514 bis 539 bzw. 529 bis 552 von EBNA1 befindet,
b) dem Peptid mit 16 Aminosäuren, dessen Sequenz sich an Positionen 68 bis 83 von LMP1 befindet, und
c) den Peptiden mit 21 und 17 Aminosäuren, deren Sequenz sich an Position 224 bis 244 bzw. 372 bis 388 von LMP2 befindet.

2. Peptid nach Anspruch 1, **dadurch gekennzeichnet, dass** die HLA 11-Moleküle eine β-Kette umfassen, die von den Allelen DRB1*0101 (HLA-DR1-Molekül), DRB1*0301 (HLA-DR3-Molekül), DRB1*0401 (HLA-DR4-Molekül), DRB1*0701 (HLA-DR7-Molekül), DRB1*1101 (HLA-DR11-Molekül), DRB1*1301 (HLA-DR13-Molekül), DRB1*1501 (HLA-DR15-Molekül), DRB3*0101 (HLA-DRB3-Molekül), DRBS*0101 (HLA-DRB5-Molekül), DP*0401 bzw. DP*0402 (HLA-DP4-Molekül) codiert wird.

3. Peptid nach Anspruch 1 oder 2, **dadurch gekennzeichnet, dass** es ausgewählt ist aus der Gruppe bestehend aus den Sequenzen SEQ ID NO: 4, 5, 6, 11, 20 und 21.

4. Polyepitopderivat des Peptids nach einem der Ansprüche 1 bis 3, **dadurch gekennzeichnet, dass** es die Sequenz des Peptids und die Sequenz wenigstens eines weiteren identischen oder anderen Epitops umfasst.

5. Polyepitopderivat nach Anspruch 4, **dadurch gekennzeichnet, dass** es ein CD4+-T-Epitop umfasst, dessen Sequenz der des Peptids nach einem der Ansprüche 1 bis 3 entspricht, und wenigstens ein weiteres Epitop, ausgewählt aus der Gruppe bestehend aus:
- einem CD8+-T-Epitop eines EBV-Antigens,
- einem B-Epitop eines EBV-Antigens und
- einem natürlichen oder synthetischen universellen CD4+-T-Epitop, wie dem TT 830-846-Peptid, PADRE, dem Peptid 45-69 des HIV-1-Nef-Proteins und den Peptiden CS.T3, CSP, SSP2, LSA-1 und EXP-1.

6. Fusionsprotein, **dadurch gekennzeichnet, dass** es aus einem Protein oder einem Proteinfragment besteht, das an ein Peptid nach einem der Ansprüche 1 bis 3 oder ein Polyepitopderivat nach Anspruch 4 oder Anspruch 5 fusioniert ist.

7. Fusionsprotein nach Anspruch 6, **dadurch gekennzeichnet, dass** das Protein oder das Fragment ausgewählt ist aus der Gruppe bestehend aus einer α- oder β-Kette eines aus den Molekülen HLA-DR1, HLA-DR3, HLA-DR4, HLA-DR7, HLA-DR11, HLA-DR13, HLA-DR15, HLA-DRB3, HLA-DRB4, HLA-DRB5 und HLA-DP4 ausgewählten HLA II-Moleküls oder der extrazellulären Domäne der einem löslichen HLA II-Molekül entsprechenden Kette.

8. Lipopeptid, **dadurch gekennzeichnet, dass** es erhalten ist durch Addition eines Lipids an eine α-Aminofunktion oder eine reaktive Funktion der Seitenkette eines Peptids nach einem der Ansprüche 1 bis 3 oder eines Polyepitopderivats nach Anspruch 4 oder Anspruch 5.

9. Polynucleotid, **dadurch gekennzeichnet, dass** es für ein Peptid, ein Polyepitopderivat oder ein Fusionsprotein nach einem der Ansprüche 1 bis 7 codiert.

10. Expressionsvektor, **dadurch gekennzeichnet, dass** er ein Polynucleotid nach Anspruch 9 unter der Kontrolle geeigneter regulatorischer Sequenzen für Transkription und gegebenenfalls Translation umfasst.

11. Wirtszelle, **dadurch gekennzeichnet, dass** sie durch ein Polynucleotid nach Anspruch 9 oder einen Expressionsvektor nach Anspruch 10 modifiziert ist.

12. Immunogene oder vakzinale Zusammensetzung, **dadurch gekennzeichnet, dass** sie wenigstens ein Peptid, ein Polyepitopderivat, ein Lipopeptid oder einen Expressionsvektor nach einem der Ansprüche 1 bis 10 und ein pharmazeutisch verträgliches Vehikel und/oder einen Trägerstoff und/oder ein Adjuvans umfasst.

13. Immunogene Zusammensetzung nach Anspruch 12, **dadurch gekennzeichnet, dass** sie wenigstens zwei der EBV-Peptide nach einem der Ansprüche 1 bis 3, vorzugsweise drei bis fünf Peptide, bevorzugt die sechs Peptide nach Anspruch 3, in Form einer Mischung aus Peptiden oder Lipopeptiden, wie sie in Anspruch 8 definiert sind, eines diese Peptide umfassenden Polyepitopderivats nach Anspruch 4 oder Anspruch 5, oder eines Expressionsvektors nach Anspruch 10, der diese Peptide oder dieses Fragment codiert, umfasst.

14. Immunogene Zusammensetzung nach Anspruch 13, **dadurch gekennzeichnet, dass** sie weiterhin wenigstens ein Peptid umfasst, das ein Epitop enthält, das ausgewählt ist aus der Gruppe bestehend aus einem EBV-CD8+-T-Epitop, einem EBV-B-Epitop und einem universellen CD4+-T-Epitop, wie sie in Anspruch 5 definiert sind.

15. Peptid, Polyepitopderivat, Lipopeptid oder Vektor nach einem der Ansprüche 1 bis 5, 8 und 10 als Impfstoff zur Prävention und/oder Behandlung einer EBV-Infektion oder einer begleitenden Tumorpathologie.

16. Verwendung eines Peptids, eines Polyepitopderivats, eines Lipopeptids oder eines Vektors nach einem der Ansprüche 1 bis 5, 8 und 10 zur Herstellung eines Impfstoffs zur Prävention und/oder Behandlung einer EBV-Infektion oder einer begleitenden Tumorpathologie.

17. Reagens zur Diagnose einer EBV-Infektion oder einer begleitenden Tumorpathologie, **dadurch gekennzeichnet, dass** es wenigstens ein gegebenenfalls markiertes oder komplexiertes, insbesondere mit markierten HLA II-Molekülen markiertes Peptid nach einem der Ansprüche 1 bis 3, in Form multimerer Komplexe umfasst.

18. Verwendung eines Peptids nach einem der Ansprüche 1 bis 3 zur Herstellung eines Reagens zur Diagnose einer EBV-Infektion oder einer begleitenden Tumorpathologie.

19. Verfahren zur Diagnose einer EBV-Infektion oder einer begleitenden Tumorpathologie, **dadurch gekennzeichnet, dass** es umfasst:
- Inkontaktbringen einer biologischen Probe des Individuums mit einem diagnostischen Reagens nach Anspruch 17 und
- Nachweis von CD4+-T-Zellen, die für eines der Latenzantigene EBNA1, LMP1 oder LMP2 von EBV spezifisch sind.

20. Verfahren zur Sortierung von CD4+-T-Zellen, die für eines der Latenzantigene EBNA1, LMP1 oder LMP2 von EBV spezifisch sind, **dadurch gekennzeichnet, dass** es wenigstens die folgenden Schritte umfasst:
- Inkontaktbringen einer Zellprobe mit markierten multimeren Komplexen, die durch die Bindung von löslichen HLA II-Molekülen mit wenigstens einem Peptid nach einem der Ansprüche 1 bis 3 gebildet sind, und
- Sortieren der an die HLA II/Peptid-Komplexe gebundenen Zellen.
